(19) Europäisches Patentamt

European Patent Office

Office européen des brevets

(11) **EP 1 185 612 B1**

(12) **EUROPEAN PATENT SPECIFICATION**

(45) Date of publication and mention
of the grant of the patent:
**25.08.2004 Bulletin 2004/35**

(51) Int Cl.⁷: **C12M 1/00**

(86) International application number:
**PCT/US2000/015524**

(21) Application number: **00936493.6**

(22) Date of filing: **05.06.2000**

(87) International publication number:
**WO 2000/075275 (14.12.2000 Gazette 2000/50)**

(54) **BIOREACTOR DESIGN AND PROCESS FOR ENGINEERING TISSUE FROM CELLS**

DESIGN EINES BIOREAKTORS UND VERFAHREN ZUR MANIPULATION VON ZELLGEWEBE

STRUCTURE DE BIOREACTEUR ET PROCEDE PERMETTANT DE RECONSTITUER DES TISSUS A PARTIR DE CELLULES

(84) Designated Contracting States:
**AT BE CH CY DE DK ES FI FR GB GR IE IT LI LU MC NL PT SE**

(30) Priority: **03.06.1999 US 137594 P**

(43) Date of publication of application:
**13.03.2002 Bulletin 2002/11**

(73) Proprietor: **The University of North Carolina at Chapel Hill**
**Chapel Hill, NC 27599-4105 (US)**

(72) Inventors:
• **MACDONALD, Jeffrey, M.**
**Chapel Hill, NC 27516 (US)**
• **WOLFE, Stephen, P.**
**NC 27516-8948 (US)**

(74) Representative:
**Luderschmidt, Schüler & Partner GbR Patentanwälte,**
**John-F.-Kennedy-Strasse 4**
**65189 Wiesbaden (DE)**

(56) References cited:
**EP-A- 0 113 328**          **EP-A- 0 909 811**
**US-A- 4 833 083**          **US-A- 5 015 585**

**Description**

**1. Field of the Invention**

The present invention relates generally to devices and processes of making and using devices for cell culture. In particular, the present invention relates to devices and processes of making and using devices for growth or maintenance of eukaryotic cells. One embodiment of the invention performs functions of the human liver.

**2. Background of the Invention and Recognition of the Problems**

Liver failure is classified into several major types, including acute liver failure, chronic liver disease, and multiorgan failure. The main etiologies of liver failure are viral hepatitis and hepatotoxicity induced by drugs and toxins. Advanced liver failure results in encephalopathy and coma, and may be fatal. Treatment focuses on stabilization of the patient until spontaneous recovery of liver function, or until liver transplantation. In the aggregate, the annual mortality attributable to liver failure exceeds 27,000 annually in the United States.

Artificial organs, which are devices made entirely of non-biological materials, have greatly advanced health care. Artificial organs and tissue substitutes, including kidney dialysis machines, mechanical respirators, cardiac pacemakers, and mechanical heart pumps have sustained many people with desperate life-threatening diseases. The utility of such artificial organs is reflected in their widespread use. Pacemakers, for example, serve admirably as substitutes for their biological analogs.

The artificial kidney, sometimes termed the kidney dialysis machine, illustrates both the benefits and shortcomings of purely artificial organs. Kidney dialysis machines are effective in removing urea, creatinine, water, and excess salts from the blood, thus partly fulfilling major roles of the natural kidney. Clearly, artificial kidneys have postponed deaths of patients in renal failure. However, kidney dialysis machines are insufficiently selective and inappropriately remove biological components, such as steroid hormones, that a functioning natural kidney does not. Consequently, dialysis over an extended period may result in bone loss, clotting irregularities, immunodeficiencies, and sterility.

The patient in hepatic failure, unlike the patient in renal failure, cannot be specifically treated. Renal dialysis, which revolutionized the treatment of renal failure, does not presently have a hepatic equivalent. Currently, the only available treatment for refractory liver failure is hepatic transplantation. Many patients in hepatic failure do not qualify for transplantation because of concomitant infection, or other organ failure. Because of organ shortages and long waiting lists, even those who qualify for liver transplantation often die while awaiting an allograft. UCLA reported that one quarter of their transplant candidates died before a liver could be obtained. Organs suitable for transplant in the pediatric age group are even more scarce (Busuttil, R. W. et al. *Ann Surg* 1987, 206, 387).

The natural liver has four major classes of biochemical functions. First, the liver biosynthesizes a wide range of proteins, including major acellular components of blood, such as serum albumin, alpha-anti-trypsin, alpha-macroglobulin, enzymes, clotting factors, carrier molecules for trace elements, and the apo-lipoproteins. The liver then releases these components to the blood circulation. The liver also maintains appropriate plasma concentrations of amino and fatty acids. Second, the liver has a major role in detoxification reactions. The liver oxidizes or conjugates many harmful external poisons, processes that usually, but not always, diminish the poisonous character of the toxins. The liver also destroys excess hemoglobin, metabolizes the porphyrm molecules of hemoglobin, and recycles the iron component. Third, waste products, such as bilirubin, are conjugated and excreted via the biliary tree. Fourth, the liver synthesizes and secretes the bile salts, which serve as detergents that promote the emulsification and digestion of lipids. The multiplicity and biochemical character of liver function vastly increase the complexity of extracorporeal hepatic support.

Bioartificial organs are artificial organs designed to contain and sustain a viable biological component. Many biological functions are even more complex than simply generating a voltage potential at regular intervals, as occurs in the simplest of pacemakers. Examples include biosynthesis of blood components and catabolic processing of deleterious agents. The liver, endocrine glands, bone marrow, and kidney are prominent in such specialized biochemical functions. Artificial organs without a biological component cannot reproduce the complex biochemical functions executed by these organs.

Historically, non-biologic artificial liver substitutes have depended on hemodialysis and hemoperfusion, but have been of very short-term and highly limited benefit (Abe, T. et al., Therapeutic Apheresis **2000**, *4*:26). In contrast to purely artificial organs, an effective liver replacement must have a biological component. The liver is the most massive organ in the human body, exclusive of distributed organs such as skin, gut, hematopoietic system, and vasculature. Sustaining a large mass of functioning liver cells in vitro presents a variety of hurdles. At least eight major problems to developing a functional bioartificial liver can be described: 1) growing or obtaining appropriate and viable cells; 2) providing for a critical minimum mass of cells; 3) supplying oxygen to the cells; 4) supplying nutrients to the cells, and removing cell waste products efficiently; 5) limiting shear forces and hydrostatic pressures, 6) inducing or sustaining a differentiated cell phenotype with the capacity for biosynthesis and biotransformation of toxins; 7) maintaining sterility; and 8) preventing liver tissue rejection or lysis by complement.

1) *Growing or obtaining appropriate and viable cells.* Liver cells for potential use in bioartificial livers can be established cell lines, primary isolates from human or animal livers, or primordial liver cells however, secretion of tumorigenic factors is negatively affecting FDA approval of BAL designs incorporating cell lines (Xu, A.S.L. et al., 2000 in *Lineage Biology and Liver,* Lanza, R.P., Langer R., and Vacanti, J. (Ed.), Academic Press, San Diego, pp. 559-597). Cell lines of liver are available, for example HepG2 and C3A, that express many functions of differentiated liver. Cell lines offer the potential of growing sufficient numbers of cells in an extracorporeal mass cell culture system, or bioreactor, for sustaining a patient because the growth of cell lines is not limited by cell senescence, but by nutrient availability. Primary human or animal liver cells can also be obtained in the numbers required for a functional bioartificial liver. However, the use of human liver for cell preparation is limited by its lack of availability, and the use of animal liver for cell preparation suffers from some degree of cellular incompatibility. Acute cellular incompatibility results from the binding of antibodies that recognize foreign cells followed by the binding of proteins of the complement system and lysis of the foreign cells. Longer-term cellular incompatibility mechanisms also exist, but should not present any problems for the use of bioreactors as interim or "bridge" medical products. A possible alternative to initial inoculation with a large mass of differentiated cells is the expansion of liver stem cells that are progenitors of mature liver cells. Recent reports suggest that liver progenitor cells go through multiple cell divisions on the path toward maturation and differentiation (Brill, S. et al., *Differentiation* **1995**, *59*, 95; Sigal S.H. et al., *Differentiation* **1995**, *59*, 35). Suitable control of the growth and differentiation processes with staged application of appropriate cytokines can permit preparation of a clinically useful quantity of cells.

*2) Providing for a critical minimum mass of cells.* The adult human liver has a mass of about 1400-1600 grams, and features a considerable reserve, or redundant, capacity. It is estimated that human survival can be sustained with about 15-20% of the total liver mass. The figure of 20% of the liver mass corresponds to about $5 \times 10^{10}$ cells (Kasai at al. *Artif Organs* **1994,** *18*, 348). Most, if not all, previous bioartificial liver designs suffer from a woefully inadequate cell capacity. That is, such devices are capable of sustaining far fewer than $5\times10^{10}$ cells, often orders of magnitude fewer cells. Without the cell mass critical for biosynthesis of plasma components and detoxification reactions, these other designs have little clinical utility.

3) *Supplying oxygen to the cells.* The functional units of most organs such as nephron, acinus, alveoli, microvilli, skin, etc. consists of a capillary bed across which is a physico-chemical gradient These gradients are controlled by mass transfer effects. Oxygen is the primary nutrient that is limiting in cell cultures (Macdonald, J.M. et al. *NMR Biomed* **1998**, *11*, 1; Glacken M.W. et al. *Ann NY Acad Sci* **1983**, *413*, 355). 'Integral' oxygenation, or aeration inside the bioreactor containing the biological or chemical material of interest, greatly enhances mass transfer of oxygen and carbonic acid. The formation of the latter can be used to control pH.

EP-A-0 113 328 discloses a bioreactor for maintaining cells in state of proliferation and continuous secretion of products which comprises (i) a cylindrical housing defining a chamber and having an inlet and an outlet for supply of an oxygen-containing gas integral to it, (ii) one first liquid-permeable hollow tube for supplying nutrient medium accomodated therein, (iii) one second liquid-permeable hollow tube with different permeability for exhausting cell products disposed coaxially around the first tube, (iv) a gas-permeable tube disposed in the chamber and coiled around both coaxial tubes; and (v) a compartment defined in the chamber by the space which is not occupied with tubes, wherein a semi-rigid matrix is provided to retain the cells.

US-A-4,833,083 discloses a bioreactor which comprises (i) a housing, and (ii) a plurality of modules of porous tubular members wherein each module comprises two porous tubular members wherein each module comprises two porous tubular members arranged coaxially.

However, neither the bioreactor of EP-A-0 113 328 nor the bioreactor of US-A-4,833,083 are provided with any means for regulating the flow of liquids flowing through the different compartments of the module.

EP-A-0 909 811 discloses a bioreactor for culturing human hepatocytes which comprises (i) a housing having at least one inlet and one outlet, (ii) a hollow cylindrical macroporous structure disposed therein which inner and outer surface is covered with two semipermeable membranes; thus, said membranes are arranged coaxially and define three annular spaces inside the housing, and (iii) two ports to introduce cells into the macroporous structure. This bioreactor comprises only one hollow fiber module, and the housing is provided neither with any means for introduction/expiration of gas, nor with any means for regulating the flow of liquids flowing through the different compartments of the module.

Oxygen is generally the limiting nutrient in hollow fiber bioartificial livers (Catapano, G. et al. *Int J Art Organs* **1996**, *19*, 61) primarily because hepatocytes are highly aerobic cells which causes problems of oxygen mass transfer. Oxygen has a relatively high diffusion coefficient and its mass transfer from blood in the liver sinusoids to hepatocytes is dominated by diffusion rather than convection (i.e., convection and perfusion are caused by pressure gradients). These effects are because an oxygen molecule is much smaller than other nutrients such as a glucose molecule, or than biosynthetic products such as proteins, and because the hepatocytes generate steep concentration gradients in bioartifical livers. With known rates of oxygen diffusion and oxygen consumption, and reasonable estimates of cell density, the diffusion distance at which oxygen utilization becomes the rate-limiting factor for growth is approximately 200 μm (Macdonald, J.M. et al., **1999,** in *Cell Encapsulation Technology and Therapeutics,* Kuhtreiber, W., Lanza, R.P. and Chick, W.L. (Eds.) Birkhauser Boston, Cambridge, pp. 252-286. In bioartificial livers with serial oxygenation aerated with air, oxygen becomes axially limiting in perfusion media by 25 mm (Macdonald et al., **1999,** supra).

Hepatocytes have a high metabolic rate and require a continuous oxygen supply. The oxygen consumption rate ranges from 0.59 to 0.7 nmole/s/$10^6$ cells for HepG2 cells (Smith, M.D. et al *Int J Artif Organs* **1996,** *19*, 36) and is 0.42 nmole/s/$10^6$ cells for isolated hepatocytes (Rotem, A. et al. *Biotech Bioeng 1992, 40,* 1286). Integral oxygenation, that is, continuous supply of oxygen along the path of media supply to the cells, is essential to supplying oxygen to liver cells. Serial oxygenation, which is oxygenation at one or a few places in the fluid line of media supply cannot sustain the mass of liver cells needed for an effective bioartificial liver. A difficulty with serial oxygenation is that the solubility of oxygen in aqueous media unsupplemented with oxygen carriers is so low that any oxygen present is quickly depleted by cell metabolism. In fact, in longitudinal flow along a conventional bioreactor semipermeable membrane, hepatocytes deplete oxygen within 2.5 centimeters along the path and therefore convective oxygen mass transfer via increasing Starling flow is improved. Increasing flow rates through conventional bioreactors can cause fiber breeches and adversely affect hepatocyte function (Callies, R. et al., Bio/Technology **1994** *12*:75). Thus, bioartificial liver designs that do not provide for adequate oxygen delivery are able to support only a limited number of cells. In addition, the flux of oxygen in a diffusion-limited system constrains cells to grow very near (less than about 0.2 mm) to the supply of oxygen. For example, U.S. Patent No. 5,622,857 to Goffe discloses a bioreactor with some coaxial and some parallel semi-permeable hollow fibers. The Goffe design allows integral oxygenation but does not constrain the thickness of the cell compartment. The fiber-to-fiber spacing in that design is 3-5 mm so that there is not strict control of the oxygen diffusion distance. Similarly, U.S. Patent No. 5,183,566 to Darnell et al. discloses a bioreactor with bundles of hollow fibers in parallel. The Darnell et al. design does not permit a multitude of individual multi-coaxial fiber bundles to be built-up with accurate and reproducible diffusion distances, and the design is not easily scaled-up. The Darnell et al. design uses bundles of parallel fibers, again not effectively addressing the issue of oxygen diffusion. Thus, a need remains for a bioreactor which deals effectively with the diffusion-limited thickness of the cell mass, with providing a critical mass of cells, and with supplying oxygen throughout the length of the bioreactor without adverse shear force effects.

*4) Supplying nutrients to the cells, and removing cell waste products efficiently.* The issue of supplying nutrients such as carbohydrates, lipids, minerals, and vitamins has been successfully solved by several variants of hollow fiber technology, and these features must be successfully incorporated into any viable bioartificial liver or bioartificial organ design. Similarly, the issue of removing metabolic wastes is usually handled by the same system that supplies the nutrients. The consumption rates for glutamate, pyruvate, and glucose are typically in the range of 0.03 to 0.3 nmol/s/$10^6$ cells, with reasonable assumptions for cell density and growth rate (Cremmer, T. et al. *J Cell Physiol* **1981***, 106, 99;* Imamura, T. et al. *Anal Biochem* **1982,** *124,* 353; Glacken, M. Dissertation **1987).** The diffusion rates of oxygen in tissue are similar to those of pyruvate in water, and higher than those of glucose. As these consumption rates are less than the oxygen consumption rate, oxygen is the limiting nutrient in most conditions.

5) *Limiting shear forces and hydrostatic pressure.* For a given bioreactor there is an optimum balance of convection and diffusion for adequate oxygen mass transfer without creation of severe oxygen gradients. For example, using a nontoxic oxygen range, <0.4 mM (solubility constant is 1.06 mM/atm, for air solubility is 0.2 mM at 37 °C), the convective component of oxygen mass transfer should be increased as cells are increasingly farther than 0.2 mm from supply of oxygen (Macdonald et al., **1999,** supra.). Although the partial oxygen tension in the liver sinusoid is about 70 mm Hg near the portal triad dropping to 20 mm Hg near the central vein, which equates to a range of 0.096 to 0.027 mM of free oxygen, the hemoglobin-bound oxygen ranges from 6.26 to 2.91 mM. The velocity of blood flow in the liver sinusoid is about 0.02 cm/s while the oxygen diffusion coefficient is about 4 orders-of-magnitude less, or $2 \times 10^{-6}$ cm²/s. However, hepatic function is adversely affected with increasing shear forces, and *in vivo* hepatocytes are protected by a layer of endothelia and extracellular matrix in the space of Disse. Sufficient shear forces will kill hepatocytes. Others have found that shear forces induce specific cytochrome P450's (Mufti N.A. and Shuler, M.L., *Biotechnol. Prog.,* **1995,** *11,* 659). A recent study has shown that liver regenerates faster with 90% than with 70% hepatectomy and this was attributed to greater shear forces (Sato, Y. et al., *Surg. Today,* **1997,** 27, 518). However, this faster regeneration could also be due to enhanced oxygen, nutrient, and agonist mass transfer. Therefore, there is some maximum level of shear force that hepatocytes can sustain while still displaying optimal function. This maximum level can be increased if a layer of endothelia protects hepatocytes.

To increase convection, hydrostatic pressure gradients are increased. Elevated hydrostatic pressures can implode hepatocytes. Therefore, it is important to stay below these pressures. It is possible to cause 100% mortality of isolated rat hepatocytes by generating hydrostatic pressures of greater than 7 psi (>300 mm Hg) for longer than 2 minutes while inoculating these cells into coaxial bioreactor using a syringe.

*6) Inducing or sustaining a differentiated cell phenotype with the capacity for biosynthesis and biotransformation of toxins.* The use of the differentiated phenotype of liver cells is necessary to produce a useful bioartificial liver because the specialized functions of the liver, including biosynthesis of blood components and detoxification of toxins, are associated with the differentiated phenotype. These specialized functions are lost in whole, or in part, as the cells dedifferentiate, which often happens in isolated primary cell culture. In contrast, the form of liver cells capable of rapid growth is the dedifferentiated phenotype, leaving the practitioner to balance two opposing needs (Enat, R. et al. *Proc Natl Acad Sci USA,* **1984,** *81,* 1411). Some reports suggest that the phenotype of liver cells may be modulated by the presence of cytokines and extracellular matrix components. In particular, the extracellular matrix components rich in collagen IV and laminin, produced by the Engelbrech-Holm Sarcoma (EHS) cells and available commercially as MATRIGEL^TM, when used with hormonally defined media induces a differentiated phenotype (Enat, R. et al., supra; Bissell, D. M. *Scan J Gasterenterol-Suppl* **1988***, 151,*1; Brill, S. et al. *Proc Soc Exp Biol Med* **1993***, 204,* 261).

*7) Maintaining sterility.* The implementation of facile sterilization procedures for bioreactors and associated components is essential for clinical utility of extracorporeal bioartificial organs. Fortunately, the procedures for sterilization are well established, including standard methods both for sterilization of extracorporeal devices and for maintaining asepsis by standard in-line filters.

*8) Preventing liver tissue rejection or lysis by complement.* Rejection of foreign tissue may occur by a rapid process known as complement-mediated lysis that involves binding of circulating antibodies to the foreign cell surface, attachment of the proteins of the complement system, and lysis of the offending cell. The cell-mediated immune system is responsible for delayed rejection reactions. However, the cell-mediated immune system should not play a major role in bioreactor systems that do not permit direct contact of host and donor cells. Foreign body reactions, for example, against the structural components of bioreactors, are also cell-mediated and should therefore not constitute substantial obstacles.

*Needed improvements.*

In view of the above, a clear need exists for bioartificial livers to sustain patients in liver failure. Specifically, a need exists for an improved version of a bioartificial liver that would have a high biological cell capacity in very thin layers of cells, readily accessible to oxygen and nutrients. There is a need for an apparatus or bioreactor, that provides efficient oxygen delivery to large masses of cells in cell culture and permits transfer of beneficial biosynthetic cell products to the patient. Similarly, there exists a need for effective methods of use of such an apparatus.

The problems with existing bioreactor designs include inadequate oxygenation, minimal capacity for the biological cell component, limited capability for removal of toxins, excessive shear and hydrostatic forces, and difficulty in transferring biosynthetic cell products for patient use. In addition, existing bioreactor designs have not dealt effectively with the diffusion-limited thickness of the cell mass, with providing a critical mass of cells, and with supplying oxygen throughout the length of the bioreactor.

## 3. Summary of the Invention

It is therefore an object of the present invention to provide varying embodiments of an apparatus which provides efficient oxygen delivery to large masses of cells in a bioreactor cell culture and methods of use therefor.

It is a further object of the present invention to provide an apparatus which permits cells to be contained in a thin annular space adjacent to continuously oxygenated and flowing nutrient medium that provides essential oxygen and nutrients and carries away metabolic products.

It is a still further object of the present invention to provide an apparatus for the collection of the biosynthetic products of large masses of cells in a bioreactor.

It is a still further object of the present invention to provide an apparatus for the assembly of a device to detoxify blood or plasma from a patient unable to remove or inactivate these toxins.

It is a still further object of the present invention to provide an apparatus to serve as a substitute liver.

The present invention provides a scaled-up multi-coaxial hollow fiber bioreactor, having: a housing having an inner side; and an array of about 20 to about 400 modules of hollow fibers, each module having at least three coaxial semipermeable hollow fibers. The module of hollow fibers has a first fiber nested within a second fiber, the second fiber nested within a third fiber, and so on. Each fiber has an inner side and an outer side. A first compartment is defined by the inner side of the first fiber, and has at least one first inlet port and at least one first outlet port. A second compartment is defined by a first annular space between the outer side of the first fiber and the inner side of the second fiber, and has at least one second inlet port and at least one second outlet port A third compartment is defined by a second annular space between the outer side of the second fiber and the inner side of the third fiber, and has at least one third inlet port and at least one third outlet port; and successive compartments are defined by the adjacent fibers. An outermost compartment for permitting integral aeration is defined by an annular space between the outer side of the outermost fiber and the inner side of the housing, and has at least one outermost inlet port, and at least one outermost outlet port The housing has at least one inlet manifold and at least one outlet manifold for each compartment.

An additional embodiment of the present invention features a serially-linked bioreactor with multiple scaled-up multi-coaxial hollow fiber bioreactors in which two or more compartments are connected in a continuous and serial manner. This implementation is particularly useful for both the biotransformation of toxins in patient plasma and the biosynthesis of plasma components to supplement patient blood.

A further embodiment of the present invention includes a one-sided multi-coaxial hollow fiber bioreactor that is particularly adapted to NMR studies and uses in which access to all ports from one side or one end is necessary.

Yet a further embodiment of the present invention includes a two-sided multi-coaxial hollow fiber bioreactor that is particularly adapted to small scale investigations.

A still further embodiment of the present invention includes a tight-packed hollow fiber bioreactor. This implementation is particularly useful for high density culture of cells in a compact arrangement and is suitable for both the transformation of toxins in plasma and the biosynthesis of plasma components to to be used as a supplement of patient blood.

The bioreactor of the present invention, when used as a bioartificial liver, has a modular design to allow an easy adjustment in liver functional capacity depending on the weight of the patient, whether that patient is infant, child, adolescent or adult, man or woman, and on the degree of remaining liver function in the patient. The bioreactor of the present invention further has both plasma and nutrient medium compartments to permit the biotransformation of toxins in the patient plasma and to enable means of the bioreactor to enhance the effective transfer of biosynthetic products from the bioartificial liver to the patient. When used with liver or other cells, this invention is useful in the preparation of biosynthetic products for patients, in experimental use, and use as a supplemental biotransformation apparatus for detoxification of blood. The toxins in the blood may include, but are in no way limited to, metabolic wastes, products of cell or erythrocyte break-down, overdoses of ethical pharmacologic agents such as acetaminophen, and overdoses of illicit pharmacologic agents. Ease of manufacture of the invention enables cost-effective commercial development.

There has thus been outlined, rather broadly, the more important features of the invention in order that the detailed description thereof that follows may be better understood, and in order that the present contribution to the art may be better appreciated. There are, of course, additional features of the invention that will be described hereinafter and which will form the subject matter of the claims appended hereto.

As such, those skilled in the art will appreciate that the conception, upon which this disclosure is based, may readily be utilized as a basis for the designing of other structures, methods and systems for carrying out the several purposes of the present invention.

These together with other objects of the invention, along with the various features of novelty which characterize the invention, are pointed out with particularity in the claims annexed to and forming a part of this disclosure. For a better understanding of the invention, its operating advantages and the specific objects attained by its uses, reference should be had to the accompanying drawings and descriptive matter in which there is illustrated preferred embodiments of the invention.

### 4. Brief description of the drawings

Figure 1A illustrates a partial view of a multi-coaxial fiber unit comprising a plurality of compartments.
Figure 1B illustrates a graphical representation of radial oxygen concentrations.
Figure 1C illustrates a graphical representation of axial oxygen concentrations.
Figure 2A illustrates an elevation view of a multi-coaxial fiber bioreactor.
Figure 2B illustrates an enlarged view of a detail of a multi-coaxial fiber unit.
Figure 3A illustrates a set of components for centering the fibers.
Figure 3B illustrates a detail of the spacer.
Figure 3C illustrates the fiber clips.
Figure 3D illustrates a detail of the arrangement of manifolds for the bioreactor.
Figure 3E provides an alternative illustration of the manifolds.
Figure 4 illustrates the four main steps for two procedures used to construct multicoaxial bioreactors.
Figure 5 illustrates a general process of thermoplastic welding hollow fibers.
Figure 6A illustrates a perspective view of a two sided embodiment of a bioreactor.
Figure 6B illustrates a detail of the front side of the first manifold.

Figure 6C illustrates a detail of the back side of the first manifold.

Figure 6D illustrates an exploded view of the first manifold and associated elements.

Figure 6E illustrates detail of the front side of the second manifold.

Figure 6F illustrates a detail of the front side of the second manifold.

Figure 6G illustrates a detail of the back side of the second manifold.

Figure 6H illustrates a collar and a detail of the manifold.

Figure 6I is a cross-section of the second manifold.

Figure 6J illustrates a detail of the front-side of the third manifold.

Figure 6K illustrates a front perspective of the third manifold.

Figure 6L illustrates a back view of the third manifold.

Figure 6M illustrates a cross-section of the second and third manifolds.

Figure 6N provides a plan view of the complete assembly of a two sided bioreactor.

Figure 7 illustrates a stand for mass producing bioreactors.

Figure 8 illustrates a perspective view of a one sided bioreactor.

Figure 9 illustrates a small one-sided bioreactor with integral oxygenation.

Figure 10A further illustrates a serially linked bioreactor with an elevation view.

Figure 10B illustrates radial flow of the serially-linked bioartificial liver with a detail view.

Figure 10C illustrates radial flow of the serially-linked bioartificial liver with a detail view.

Figure 11A illustrates the variables used in the implementation ofDarcy's law.

Figure 11B illustrates a relationship between radial flow rate and a pressure differential.

Figure 11C illustrates a relationship between a radial flow rate and pressure in one cell compartment.

Figure 12 illustrates the algorithm for the selection of hollow fiber characteristics for attaining superior physiological function and cell viability.

Figure 13A illustrates a cross section of a multi-coaxial bioreactor.

Figure 13B illustrates a cross section of an alternative embodiment of a multi-coaxial bioreactor.

Figure 14 illustrates perspective view of an embodiment of a bioreactor.

Figure 15 illustrates a perspective view of a device used in the construction of a bioreactor.

## 5. Description of the Preferred Embodiments

### 5.1 Definitions

*Annular space.* The radial distance separating two adjacent hollow fibers.

*BAL.* Bioartificial liver. Also, specific embodiments of the present invention: the scaled-up multi-coaxial hollow fiber bioreactor, the tight packed hollow fiber bioreactor or the serially-linked bioreactor with a complement of liver cells, nutrient medium, and gases.

*Bioreactor module.* Coaxially-arranged semipermeable hollow fibers. One module forms the core of the multi-coaxial hollow fiber bioreactor whereas the scaled-up multi-coaxial hollow fiber bioreactor comprises many modules.

*Biotransformation.* The metabolic detoxification of blood or plasma by tissues or cells.

*Fourth compartment.* The compartment, if present, in a bioreactor embodiment that is bounded by the outside of the third hollow fiber and the inside of the fourth, that is, adjacent, hollow fiber, and is connected to two ports, *the fourth compartment inlet port* and *the fourth compartment outlet port.*

*First compartment.* The compartment in any of the bioreactor embodiments that is bounded in part by the inside of the first and innermost coaxial hollow fiber and is connected to two ports, the *first compartment inlet port* and *the first compartment outlet port.*

*Integral aeration.* Exposure to a gas, typically air or oxygen with carbon dioxide, at almost all points along a flow path. Integral aeration is distinguished from *serial aeration,* in which a bubbler or gas exchange device is inserted at one point in the fluid circuit.

*Manifold.* A part of the bioreactor located at an end of the fibers and intended to physically separate compartments and split flow of fluids.

*Microfiber or microbore hollow fiber.* A semipermeable hollow fiber of 200 to 500 micrometer o.d.

*Multi-coaxial hollow fiber bioreactor.* The bioreactor comprising three or more coaxially-arranged semi-permeable hollow fibers encased by a hollow housing.

*Nutrient medium.* The balanced electrolyte solutions enriched with sugars, trace minerals, vitamins, and growth enhancers. Each particular formulation is named by or for the formulator, sometimes with whimsical or non-illuminating designations. Nutrient media include, but are not limited to: RPMI 1640 (Roswell Park Memorial Institute, formulation #1640), Ham's F-12 (the twelfth formulation by Dr. Ham in his F series), DMEM (Dulbecco's modified Eagle's medium), and CMRL-1415 (Connaught Medical Research Laboratory formulation #1415). Nutrient media are routinely enhanced by addition of hormones, minerals, and factors known to those of ordinary skill in the art, including, but in no way limited to, insulin, selenium, transferrin, serum, and plasma.

*One-sided multi-coaxial hollow fiber bioreactor.* The version of the multi-coaxial hollow fiber bioreactor that has both inlet and outlet ports on the same end plate. This version is particularly adapted to NMR studies and to studies where access to all ports from one side is necessary.

*Outermost compartment.* The compartment in any of the bioreactors that is bounded by the outside of the outermost hollow fibers and the inside of the housing, and is connected to two ports, the *outermost compartment inlet port* and the *outermost compartment outlet port.*

*Potting.* A term of the art meaning the joining of elements, as by gluing, or any other suitable means.

*Scaled-up multi-coaxial hollow fiber bioreactor.* The bioreactor comprising arrays of from about 20 modules to about 400 modules of coaxially-arranged semi-permeable hollow fibers, where the entire set of modules is encased by a hollow housing.

*Second compartment.* The compartment in a bioreactor embodiment that is bounded by the outside of the first and innermost coaxial hollow fiber and the inside of the second, that is, adjacent, coaxial hollow fiber, and is connected to two ports, the *second compartment inlet port* and the *second compartment outlet port.* In the one-sided multi-coaxial hollow fiber bioreactor and in some dead-ended fiber designs only one port provides access to compartment 2.

*Serially-linked bioreactor.* The system comprising a plurality of scaled-up multi-coaxial hollow fiber bioreactors or of tight-packed hollow fiber bioreactors, or a combination, in which two or more compartments are connected in a continuous and serial manner. In this context, each scaled-up bioreactor is referred to as a bioreactor subunit.

*Third compartment.* The compartment in any of the bioreactor embodiments that is bounded by the outside of the second hollow fiber and the inside of the third, that is, adjacent, coaxial hollow fiber, and is connected to two ports, the *third compartment inlet port* and the *third compartment outlet port.*

*Tight-packed hollow fiber bioreactor.* The scaled-up bioreactor comprising arrays of from about 20 modules to about 400 modules of coaxially-arranged semi-permeable hollow fibers. Microfibers for aeration are arranged parallel and adjacent to the modules and the whole encased by a hollow housing.

### 5.2 Elements of the Apparatus

The instant invention includes a modular multi-coaxial bioreactor, having in theory, no limit to the number of coaxial fibers. In a preferred embodiment a scaled-up multi-coaxial bioreactor comprises at least two sets of manifolds, at least three hollow fiber sizes, at least two sets of end caps, and a housing. This embodiment of the bioreactor contains at least four separated compartments. The modular design is composed of two sets of manifolds, with each pair of manifolds connected to each end of the fibers. There is a series of about 20 to about 400 holes coaxially arranged across the sets of manifolds and coaxially aligning the fibers. The manifolds optionally include flow distributors so that fluid and gas phase flow rates through the fibers are approximately uniform. The fiber manifold assemblies are attached radially from the largest to the smallest diameter fibers, and axially from the smallest to the largest diameter fibers. Fibers with smaller diameter are inserted into fibers of larger diameter and the respective manifolds are sealed together. To align fibers, two or more dowels are inserted through precisely drilled holes in the three manifolds or by interconnecting tongue and grooves on adjacent manifolds.

The bioreactors of the current invention advantageously combine 'integral' oxygenation with defined diffusion distances, have ports to accommodate potential bile duct formation, and/or are easily scalable. Integral oxygenation permits efficient mass transfer of dissolved gases and control of pH. Defined diffusion distances permit predictable axial and radial physico-chemico-biological parameters such as shear forces, availability of nutrients, and pH. In use with patients, one or more of the at least four compartments can be used for patient blood plasma while another can be used to perfuse cells with integrally oxygenated media. Optionally, two or more bioreactor units are attachable in series so that toxins can perfuse out of plasma radially through the cell mass in one unit and infuse synthetic factors in the next unit. There is the potential for the biliary system to develop using the ports as the bile duct exit ports.

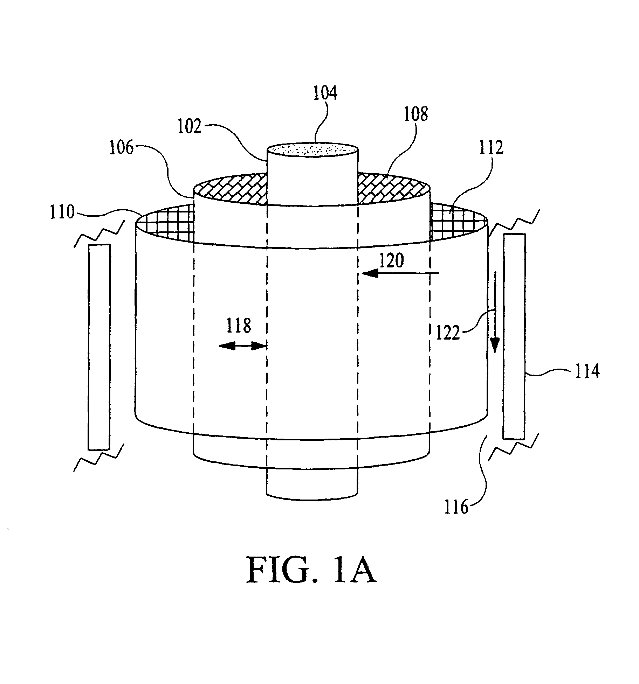

Figure 1A illustrates a multi-coaxial fiber unit according to the instant invention comprising a plurality of compartments. Innermost fiber **102** provides intracapillary space or first compartment **104** for the receipt of standard media or plasma. Middle fiber **106** provides annular space or first middle compartment **108** for the containment of cells such as liver cells. Outer fiber **110** provides extracapillary space or second middle compartment **112** for the receipt of media. Housing **114** defines the outermost perimeter of the multi-coaxial fiber unit. Space or outermost compartment **116** between housing **114** and outer fiber **110** allows for the receipt of a gas. Diffusion distance **118** is half the width of first middle compartment **108**. Radial perfusion **120** is the radial flow of, for example, dissolved oxygen through the fiber unit, while axial perfusion **122** is the axial flow of, for example, dissolved oxygen through the fiber unit.

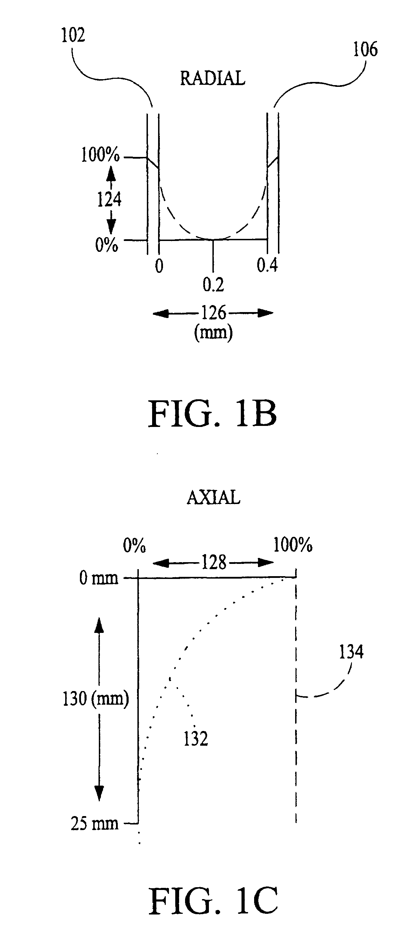

Figure 1B shows a radial oxygen gradient across the first middle compartment **108** of a coaxial bioreactor having viable cells and integral oxygenation, where oxygen concentration is equal in the first compartment **104** and second middle compartment **112,** and an annular space **126** of 0.4 mm (millimeters) separators **102** and **106.** As shown, oxygen percentage **124** approaches zero as distance **126** approaches the diffusion distance **118** of 0.2 mm. As distance approaches 0.4 mm, percentage oxygen **124** approaches 100 percent, again due to oxygen diffusion from the second middle compartment **112.**

Figure 1C shows axial oxygen gradients obtained through the use of bioartificial liver bioreactor designs having either "in-series" or "in-parallel" oxygenation. As shown, percentage of oxygen **128** decreases with distance **130** when "in series" **132** oxygenation is used, but remains nearly constant when "in parallel" **134** oxygenation is used, thereby demonstrating the decreased axial oxygen gradient due to integral oxygenation.

This invention permits precise control of desired diffusion distances in conjunction with integral oxygenation, and the modular design in conjunction with the potting material permits fibers of any composition to be arranged in a desired multi-coaxial order. Potting materials are known to those skilled in the art; such as that disclosed in U.S. Patent No. 4,227,295 to Bodnar et al. Where thermoplastic fibers are used, such as polypropylene, polyethylene, polysulfone, etc., then bioreactors can be constructed using a thermal bonding method such as described by Robinson in U.S. Patent No. 5,015,585.

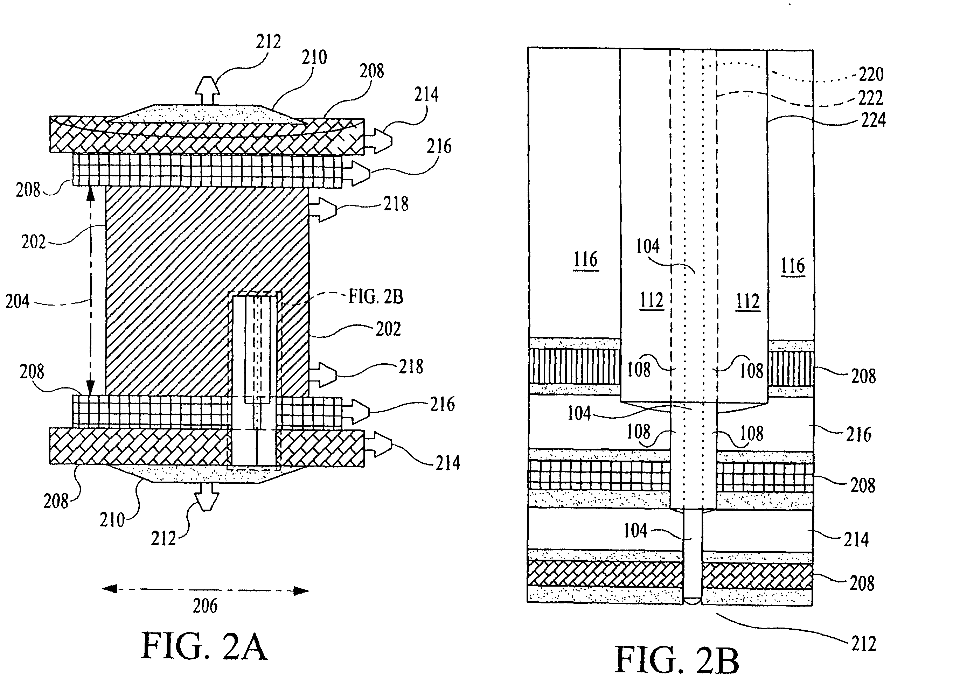

Figure 2A illustrates a scaled-up multi-coaxial bioreactor comprising a plurality of multi-coaxial fiber units. In a preferred embodiment, the bioreactor comprises from about 20 to about **400** multi-coaxial fiber modules contained in housing **202.** However, in theory, there is no limit to the number of coaxial fibers. As shown, housing **202** is 17 centimeters in length **204** with an 8 centimeter diameter **206,** although alternative dimensions are envisioned. For example, the length of fibers can be from about 2 centimeters to about 50 centimeters and the diameter can be from about 1 centimeter to about 100 centimeters. Housing **202,** for example, comprises three sets of manifolds **208**, and one set of endcaps **210,** one for each respective end of the bioreactor. One set of manifolds is needed for each size of hollow fiber. The housing is constructed of glass, polycarbonate, polypropylene, polyethylene, Delrin, Teflon, steel, brass, ceramic, or any other suitable material. Manifolds **208** and endcaps **210** can be machined or formed from acrylic, thermoplastic, ceramic, or any other suitable material. The hollow fibers comprising each multi-coaxial fiber module are preferably semi-permeable, and are in each of, for example, 3 sizes: approximately 5 millimeter outer diameter (o. d.) polysulfone; approximately 3 millimeter o.d. polysulfone; and approximately 1 millimeter o.d. cellulose acetate. There are thus three fibers per coaxial fiber unit, and three manifolds per module of fibers with the manifolds connected to each end of the fibers. The desired ΔP can also be created by restricting the effluent flow rate from port **212**, for example by use of a needle valve. The invention also envisions embodiments having more than three coaxial fibers in each fiber module, with corresponding manifolds and ports.

Each bioreactor of the above listed or similar dimensions can support up to about 160 grams of tissue. Furthermore, in another embodiment a larger cell mass capacity is supported by using a larger annular compartment for the cell compartment and/or using longer fibers. An annular design for the cell compartment has an additional advantage over using single tubes because orders of magnitude greater biomass can be obtained. For example, a bioartificial liver described by Hu et al., U.S. Patent No. 5,605,835 where hepatocytes are encapsulated in collagen in the innermost intracapillary compartment, would require 44,860 cellulose acetate hollow fibers of 200 micrometer diameter to attain the about 120 grams of tissue needed as an extracorporeal liver. In contrast, the embodiment of the present invention illustrated by Figure 2 uses a factor of 150-fold fewer fibers.

Openings leading to ports allow for the movement of materials. Innermost ports **212** allow for the flow of media or plasma through the bioreactor. First middle ports **214** allow for the inoculation of cells into, or flow of cells through, the bioreactor. Second middle ports **216** allow for the flow of media through the bioreactor. Lastly, outermost ports **218** allow for the flow of gas through the bioreactor. Alternative uses of ports are also envisioned. For example, media can flow through ports **218,** cells into, or through, ports **216,** media or plasma through **214,** and oxygen or other gases through **212.**

Figure 2B illustrates an enlarged view of a multi-coaxial fiber unit comprising a plurality of compartments located within the multi-coaxial bioreactor. As shown, media or plasma residing within first compartment **104** can either flow to or be received from innermost port **212.** Similarly: (1) cells residing within first middle compartment **108** can either flow to. or be received from first middle port **214;** (2) media located within second middle compartment **112** can either flow to, or be received from second middle port **216;** and (3) gas residing within outermost compartment **116** can either flow to, or be received from outermost port **218.** Also depicted is a module of hollow fibers consisting of innermost fiber **220**, middle fiber **222** and outermost fiber **224.** Embodiments comprising modules with more than one middle fiber are also envisioned.

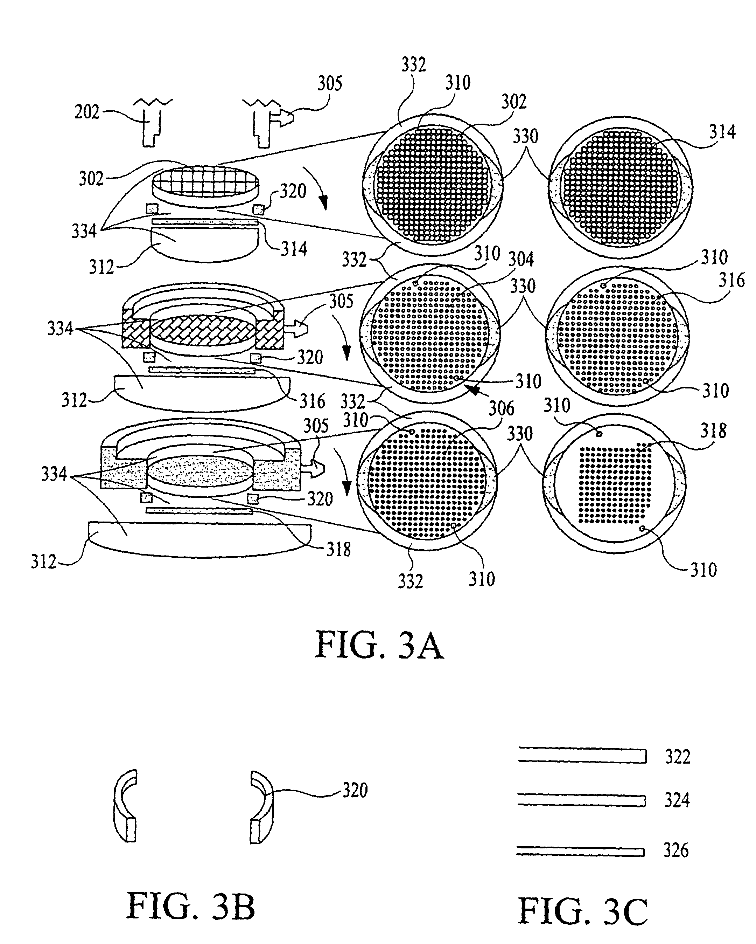

Figure 3A further illustrates a set of manifolds **208,** including first manifold **302,** second manifold **304**, and third manifold **306.** The manifolds each consist of a circular region about 15 centimeters in diameter, each with about 20 to about 400 holes, although any number of holes is possible. The holes are of three different diameters corresponding to the outer diameter of the respective fibers. This permits 20 fibers per row with a 2 mm space between holes, although optimum hole patterns are based on the number of desired fiber modules. These fibers can consist of any desired hollow fiber or tube with appropriate changes in the design specifications of the respective parts to fit the respective fiber diameters.

Two exemplary potting procedures are described: one that results in centered coaxial fibers, and one that does not. In both procedures the fibers are inserted into respective manifolds **208**. The outer fiber **110** can be composed of any air permeable material such as, silicon, ceramic, glass, etc. As an example, polypropylene outer hollow fibers can be used in conjunction with thermoplastic welding.

The potting procedure used to center the fiber requires three additional sets of parts shown in Figures 3A, 3B and 3C: (1) first hollow fiber guide **314** for first manifold, second hollow fiber guide **316** for second manifold, and third hollow fiber guide **318** for third manifold, (2) spacers **320,** and (3) hollow fibers clips (outer **322,** middle, **324,** and inner, **326)** for each respective hollow fiber size (outer **110,** middle **106,** and inner **102).** The hollow fiber guides can be composed of any material that can maintain rigidity at 3 mm thickness. The manifold and hollow fiber guide are placed against each other, sequential manifold holes are aligned with dowels, and outer fibers **110** are inserted through the holes of the manifold and then the hollow fiber guide with the ends of the fibers extending at least 1 cm beyond hollow fiber guide. The fibers are inserted in the housing **202** and the other end of the outer fibers are inserted into next first manifold **302** and first hollow fiber guide. The hollow fiber guide is extended away from manifold a distance permitting the pair of spacers **320** to slide between manifold and hollow fiber guide. The spacers **320** are glued on respective sides to adjacent manifold extensions **330**. Once manifolds **208**, spacer **320**, and hollow fiber guide are secured, the hollow fibers are secured with tape or clips. Outer **322,** middle **324,** and inner **326** fiber clips that fit each fiber size are inserted across each row of fibers serving to keep fibers taut to maintain coaxiality and to pinch close the inner lumen so epoxy will not clog hollow fiber during potting. Therefore, fiber clips are varying lengths depending on row length. The secured fibers, spacer, and hollow fiber guide are inserted into a casting pot **312** which is screwed on or sealed to manifold **208** and serves to retains the epoxy during the potting process. The assembly is placed upright with casting pot **312** on the bottom and with a 10 to 45 degree downward tilt toward port **305** potting material, e.g., epoxy, is injected into side port **305** and runs down the side of the manifold **208** through holes **332** filling voids **334** contained by casting port **312**. The bottom of the casting pot **312** is tapped to remove air bubbles. An appropriate amount of epoxy is added to fill voids **334** and to a level to cover both sides of manifold **308.** Once the epoxy has nearly cured, the hollow fiber guide, spacer **320,** casting pot **312,** and epoxy is cut off leaving a very small layer of epoxy **308** over manifold. Therefore, the spacers **320,** hollow fiber guides **314, 316, 318** and fiber clips **322, 324** and **326** serve two purposes: (1) they keep fibers taut and centered while potted, and (2) create a region for epoxy to cure between hollow fiber interstices that can be cut leaving a layer of epoxy **308** over manifolds **208.**

Other methods can be used to center the inner fiber. In a first example, a rigid rod is inserted into the lumen of the inner fiber to center it. Then cells are mixed with a temperature-dependent gelling matrix, such as collagen, and the matrix is induced to gel. Then, the rod is removed. In a second example, a monofilament fiber with a diameter equal to the necessary inter-fiber spacing acts as a spacer. The monofilament made of polypropylene, polyethylene, or other appropriate material is helically wound around the inner fiber with one complete revolution every centimeter or so. The inner fiber with its monofilament winding is then inserted into the middle fiber. This permits the inner and middle fibers to be axisymmetric. Therefore, the inoculated cells undergo a cork screw-type path. The monofilament is potted with the inner fiber using element **306.**

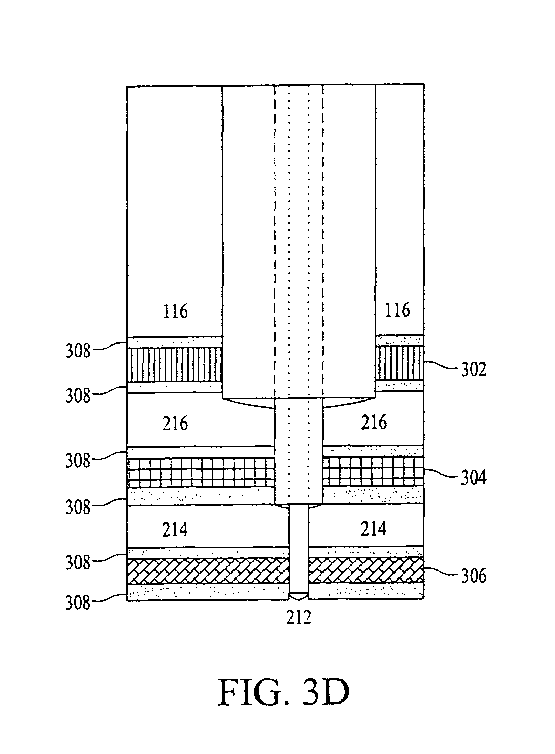

Figure 3D illustrates a cut out and enlarged view of a multi-coaxial fiber unit comprising a plurality of compartments located within the multi-coaxial bioreactor and formed by the centered coaxial fiber process. Thus, first manifold **302** is bound on the top and bottom by epoxy **308** and partially defines outermost compartment **116** and second middle port **216**. Second manifold **304** is bound on the top and bottom by epoxy **308** and partially defines second middle port **216** and first middle port **214**. Third manifold **306** is bound on the top and bottom by epoxy **308** and partially defines innermost port **212.**

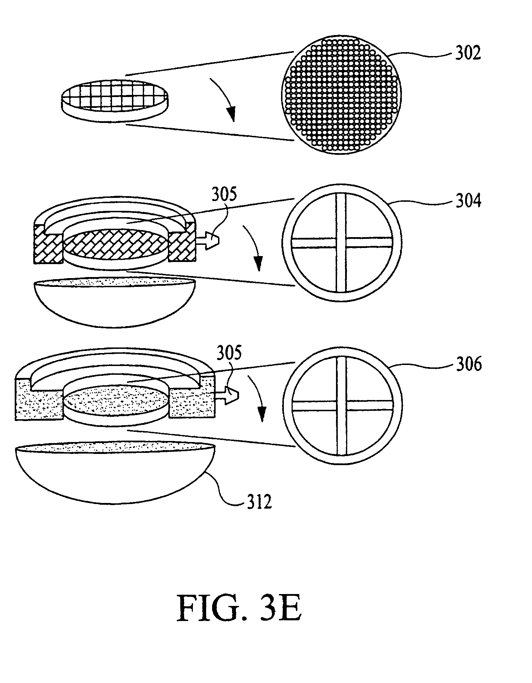

The potting process resulting in non-centered fibers does not require hollow fiber guides, monofilament, or spacers but requires a centrifuge. Figure 3E is an illustration of three sets of manifolds 208, showing first manifold **302,** second manifold **304,** third manifold **306,** and casting pot **312**. Note the second manifold **304** and third manifold **306** do not have holes and will not coaxially align fibers. The epoxy potting process is described by Bodnar et al. (U.S. Patent No. 4,227,295). This process involves inserting the fiber ends into the two respective manifolds. The fibers ends are gathered and then secured and sealed with tape, string, or some other material. Then, the casting pots **312** are attached to both ends. The assembly is attached to a centrifuge rotor at the axial center of the assembly. A small flat container is placed above the assembly, and has holes at each end which are attached to the ports **305** at each end of the assembly. As the centrifuge rotates, epoxy is placed in the middle of the container and is forced out toward the ends of the container, down the holes of the container, through the ports **305,** and into the casting pots **312** filling the interstices of the fiber bundle. Once the appropriate amount of epoxy has flowed to the casting pots **312,** the assembly continues to spin until the epoxy partially cures. Then the excess epoxy and casting pots **312** are cut away at both ends leaving open hollow fiber holes at both ends of the assembly, and the next epoxy potting step is performed. To insure that leaks do not occur between manifolds, the manifolds **208** are welded or epoxied along their borders.

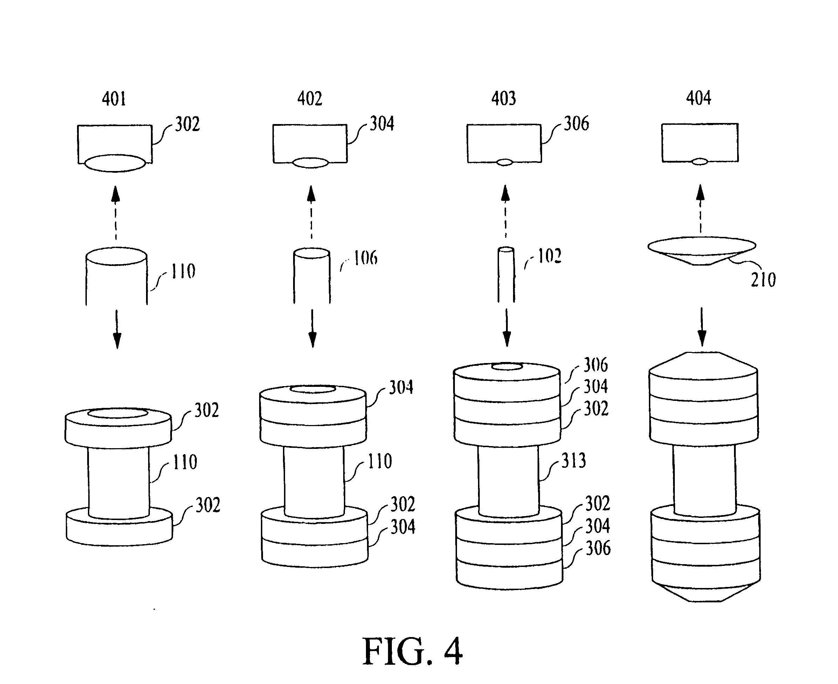

Figure 4 is an illustration describing the four main steps of both procedures for assembly of one module of the instant invention which is composed of three sets of fibers. The procedure is, first step **401** insert the outer fiber **110** ends into holes in first manifolds **302**, then attach first manifold **302** to housing **202**, and pot fiber ends. The next step **402** is insert second set of fibers, or middle fibers **106**, into outer fiber **110**, insert fiber ends in holes in the second manifolds **304**, and pot fiber ends. The next step **403** is insert inner fibers **102** into middle fibers **106** and insert fiber ends into holes on the third manifolds **306** and pot fiber ends. The last step **404** is attach endcaps. At least two dowels are inserted into adjacent manifolds during the second **402** and third **403** steps by way of dowel holes **310** to align holes in adjacent manifolds. The second manifold **304** has four dowel holes **310** because at least 2 are filled with a hollow fiber that maintains dowel hole void **310** after potting process is complete so dowel can be inserted and second manifold **304** can be aligned properly with third manifold **306.** Thermoplastic welding can be performed on similar materials, therefore, in the instant invention the first manifold **302** is made of polypropylene in order to weld polypropylene fibers.

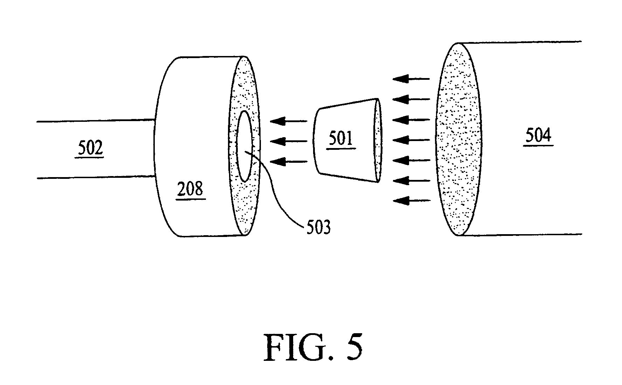

Figure 5 illustrates the general process of welding. A Teflon cork **501** is inserted in the inner diameter of the hollow fiber **502** forcing the fiber walls against the wall of the holes **503** in the manifold **208**. A heat gun, or thermoplastic welder **504** is used to melt the fiber wall **502** and wall of the holes **503** together. Once the assembly is cooled the teflon plug is removed. A jib of teflon plugs that match the first manifold **302** holes is used to weld the outer fibers ends to the respective first manifolds **302**. Both ends of the housing **313** have a slot into which first manifold **302** fits. A similar jib of teflon plugs fitting the second manifold **304** and third manifold **306** can be used to thermoplastically weld all three sets of fibers if fibers are composed of same material as manifold, and most likely will result in non-centered coaxial fibers. In the first step **401,** first manifold **302** is welded to housing **313.** In second step **402,** second manifold **304** is welded to manifold **302**. In third step **403**, third manifold **306** is welded to second manifold **304**. In fourth step **404**, endcaps **210** are welded to third manifold **306.**

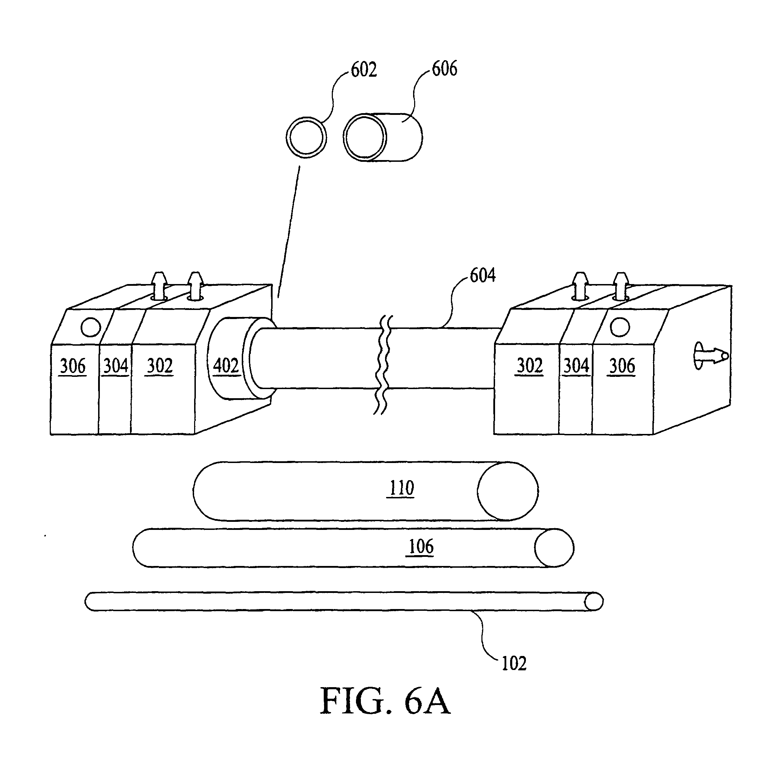

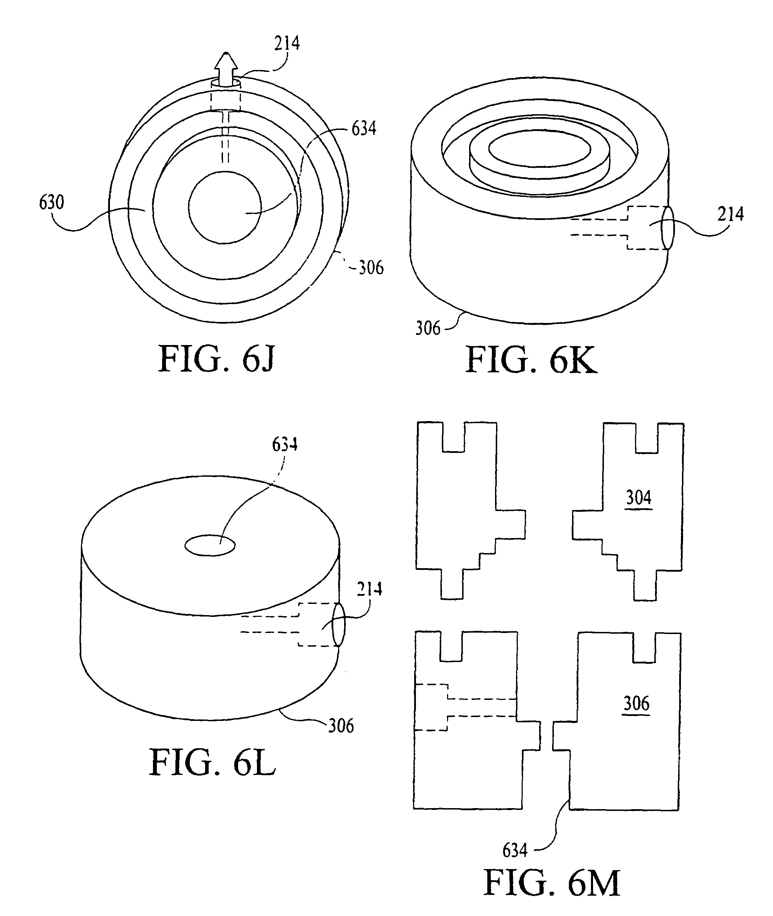

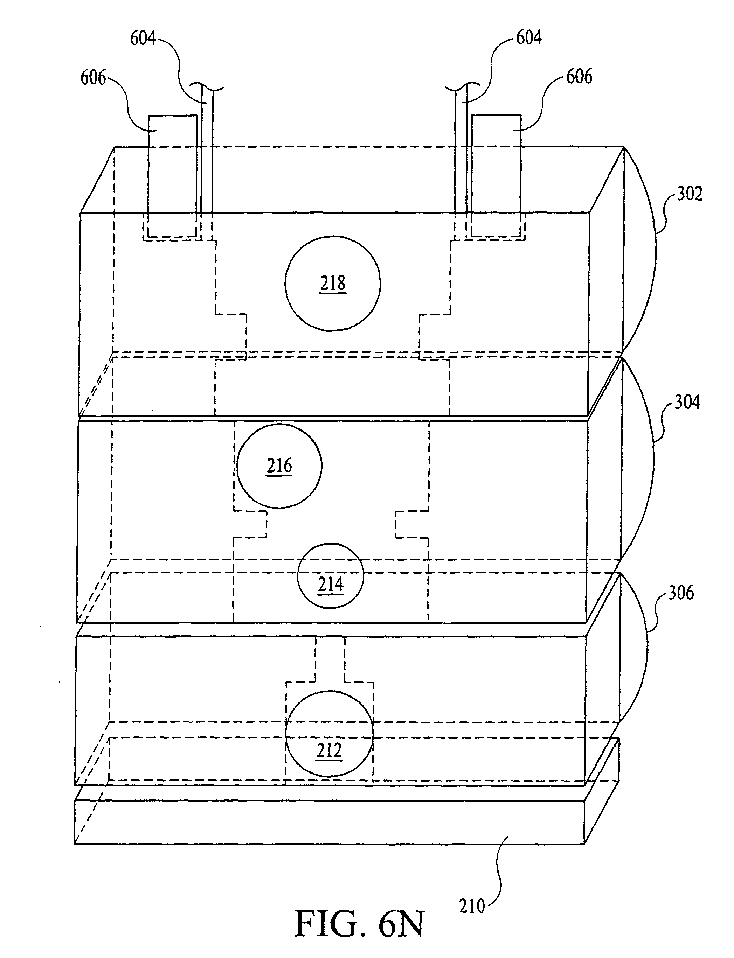

Figure 6A illustrates a two-sided multi-coaxial hollow fiber bioreactor. The bioreactor is assembled using parts machined or formed from polypropylene or any other machined or formed material using two of each part, one for each respective end of the bioreactor, three semi-permeable hollow fibers, and an approximately eight to ten millimeter diameter nuclear magnetic resonance (NMR) tube (available, for example, from Wilmad Inc., Buena, N.J.). The hollow fibers used to construct the bioreactor are approximately 8 millimeters o.d. with wall thickness of 0.5 millimeters, 2.6 millimeters o.d. with wall thickness of 0.4 millimeters, and 0.8 millimeters o.d. with wall thickness of 0.2 millimeters and made of polypropylene with 0.2 micrometer pore size (available, for example from Akzo-Nobel, Germany). The respective fibers can be composed of a 8 millimeter o.d. silicone tube (available, for example, from Dow Coming, Midland, MI), and 3 and 1.3 millimeter o.d. polysulfone hollow fibers (available, for example, from AG/Technologies, Inc., Wilmington, DE) with 0.4 and 0.2 millimeter wall thickness, respectively, and with 0.1 and 0.65 micrometer pore sizes, respectively. Alternatively, a smaller o.d. and thinner walled silicone tube can be used for the outer fiber **110** and pulled over a perforated rigid tube to give structural rigidity. Diffusion of oxygen is inversely proportional to the wall thickness, and therefore, the thinner walled silicone tubing will permit superior mass transfer of oxygen. The 2.6 millimeters o.d. polypropylene fiber can be replaced and constructed with a 3 millimeters o.d. polysulfone fiber with a wall thickness of 0.5 millimeters and with 0.65 micrometer pore size. These fibers can consist of any desired hollow fiber or tube with appropriate changes in the design specification of the respective parts to fit the respective fiber. Fibers are cut to be at least 2 millimeters longer than their final length when assembled.

The outer fiber **110** can be coated with a perfluorinated polymer to bridge or clog the pores to avoid 'wetting', or saturation with water, or pores and effectively eliminating evaporation of water from media using a process provided by Compact Membrane Systems (Wilmington, Delaware) or other similar pore-filling process. Coating could also be performed on the scaled-up multicoaxial bioreactor described in Figure 3. For mass transfer purposes, the coating occurs on the media sided of the fiber. If coating is not performed the air compartment must have higher pressure than adjacent media compartment to avoid pore wetting, and the gas stream should be warmer than media stream to decrease evaporation and condensation.

In the instant invention, the two-sided multi-coaxial bioreactor is assembled with largest outer fiber **110** (e.g., 5 millimeters i.d., 8.1 millimeters o.d.) and first manifold **302**. Securing means such as polyurethane epoxy or thermoplastic welding or combination thereof are used to fix the fibers to the respective parts. Housing, an NMR tube **604,** is secured to first manifolds **302** by collars **606** and O-rings **602**.

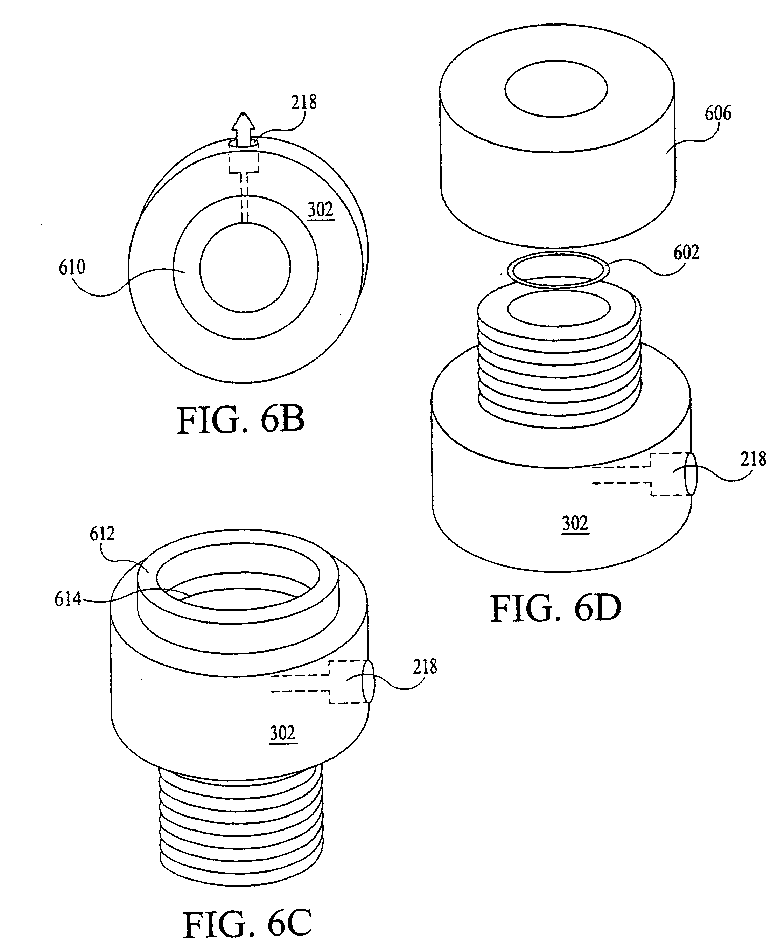

Figures 6B, 6C and 6D further illustrate the first manifold. As shown in Figure 6B, the front side comprises first circular recessed area **610** while as shown in Figure 6C the back side comprises first circular raised area **612**. Both first circular recessed area **610** and first circular raised area **612** partially define first void **614.** As shown in Figure 6D, O-ring **602** is placed into first circular recessed area **610**. Collar **606** is attached thereto. Outermost port **218** is also shown.

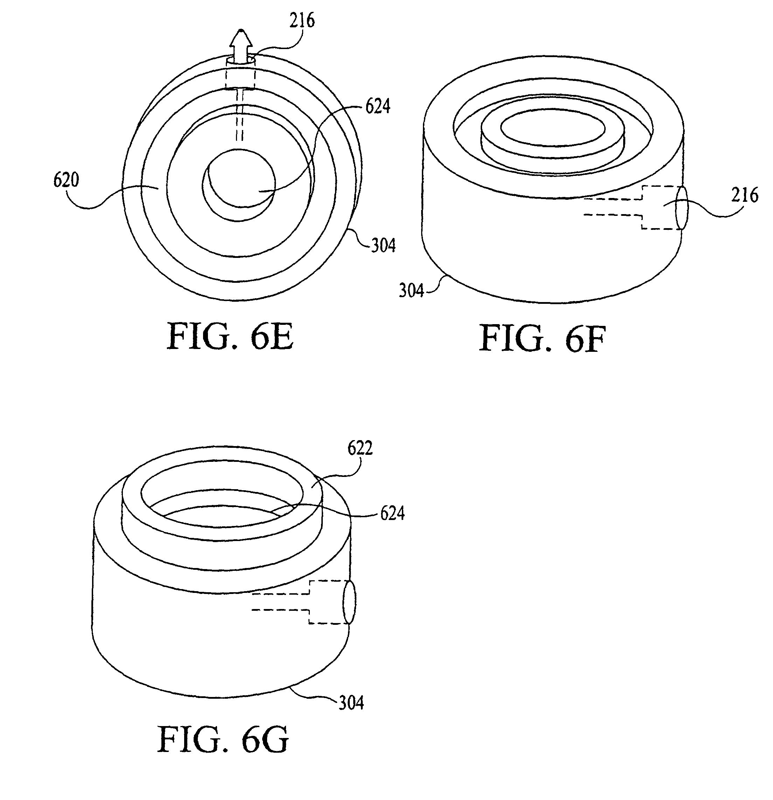

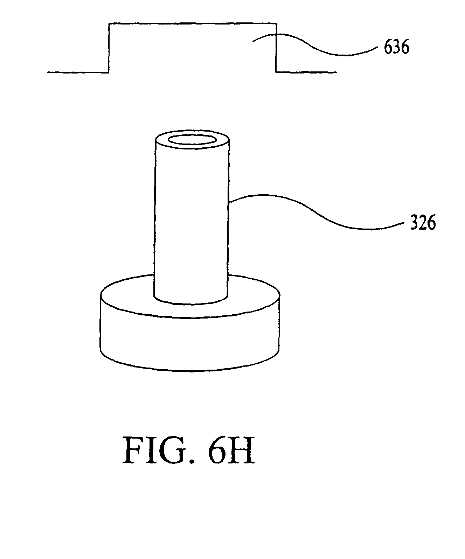

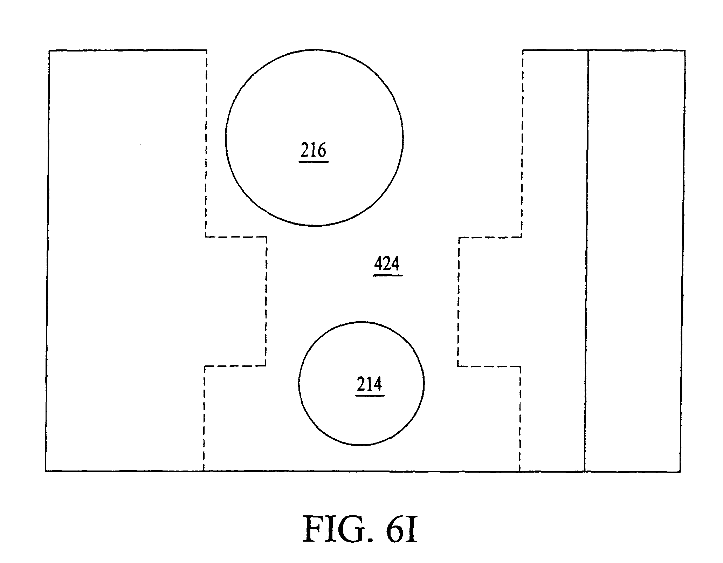

Figures 6E, 6F, 6G and 6H further illustrate the second manifold. As shown in Figure 6F, the front side comprises second circular recessed area **620** while, as shown in Figure 6G, the back side comprises second circular raised area **622**. Both second circular recessed area **620** and second circular raised area **622** partially define second void **624**. In order to insure that cells do not collect in the space between second manifold **302** and third manifold **304,** a collar **326** can be slipped over the middle fiber **106** and snuggly fitting into the second void **624** and into the front void **636** of third manifold **304.** Second middle port **216** is also shown. Figure 6C also illustrates the fashion in which first manifold **302** communicates with second manifold **304.**

Figure 6I provides a perspective view of the second manifold, illustrating second void **624,** second middle port **216**, and first middle port **214.**

Figure 6J, 6K and 6L further illustrate the third manifold. As shown in Figures 6J and 6K, the front side comprises third circular recessed area **630** that partially defines third void **634.** First middle port **214** is also shown in Figure 6L. Figure 6M also illustrates a cross section of the complete assembly, showing how second manifold **304** communicates with third manifold **306.**

Figure 6N is a top view of the complete assembly of a two sided bioreactor with all four inlet and outlet ports on one side of the manifold, illustrating first manifold **302**, second manifold **304,** third manifold **306**, media port **212**, cell port **214,** media port **216,** gas port **218** and collar **606.**

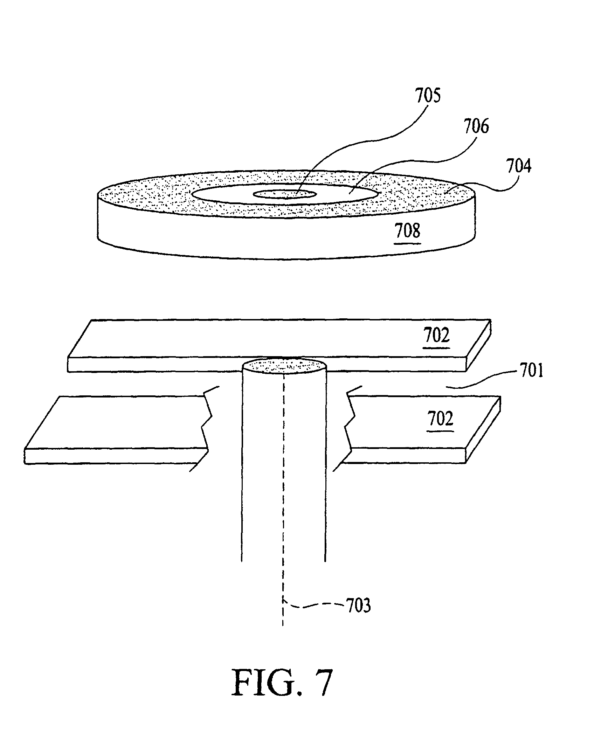

The bioreactor can be mass produced using stand or holder **702** illustrated in figure 7. The stand holds the manifold **208** and fiber axis **703** in a groove **701** so that the axis of the fiber **703** is 90 degrees perpendicular to the plane of the manifold **704** containing the fiber hole opening **705.** Then the fibers are fixed by filling the manifold/fiber space **706** with epoxy, or a thermoplastic welding process as depicted in Figure 5. If epoxy is used then the epoxy is rapidly cured in an oven and then the other side is potted and the entire procedure depicted in Figure 7 is repeated. The manufacture can be automated for mass production where the process of bioreactor assembly shown in Figure 4 is performed robotically. In a mass produced two-sided bioreactor, the O-rings **602** and collars **610,** and threads on first manifold **302** which are used to make a seal with the 10 mm glass NMR tube **604** are replaced by using a tube composed of polypropylene or appropriate material to replace the glass 10 mm NMR tube **604** and are thermoplastically welded or epoxied to create a seal. In a preferred embodiment the outer fiber **110** is welded and the two inner fibers **102** and **106** are epoxied. With respect to the coaxial fibers, the fiber manifold **208** assemblies (Figure 6) are attached radially from the largest to smallest diameter fibers, and axially from the smallest to largest diameter fibers. The outer fiber **110** is inserted into 2 O-rings **602**, 2 collars **606,** a 10 mm NMR tube **604,** and then into the first manifold **302.** The two collars are tightened, and then first manifolds **302** at both ends of the bioreactor assembly are inserted into the grooves **701** of the stand **702.** The outer fibers **110** are thermoplastically welded in place using, for example, the process described in Figure 5. The bioreactor now assuming a dumbbell shape is ready for attaching the second manifold **304.**

A second fiber of about 2.6 millimeter o.d. polypropylene or 3 millimeter o.d. polysulfone is inserted into the outer fiber of dimension 5 mm o.d. and affixed to second manifold **304** by the epoxy process depicted in Figure 7. First manifold **302** and second manifold **304** are welded along their borders. A third fiber of 0.8 millimeter o.d. is inserted into the second fiber of 1.8 millimeter i.d. polypropylene or 2 millimeter i.d. polysulfone hollow fibers and is affixed to third manifold **306** as described above. During attachment of fibers to the manifold on only the second end of the bioreactor, the fibers are cut flush with their respective manifolds or part numbers. Endcap 210, or threaded hose bard, is glued to, or screwed onto third manifold **306** at each end.

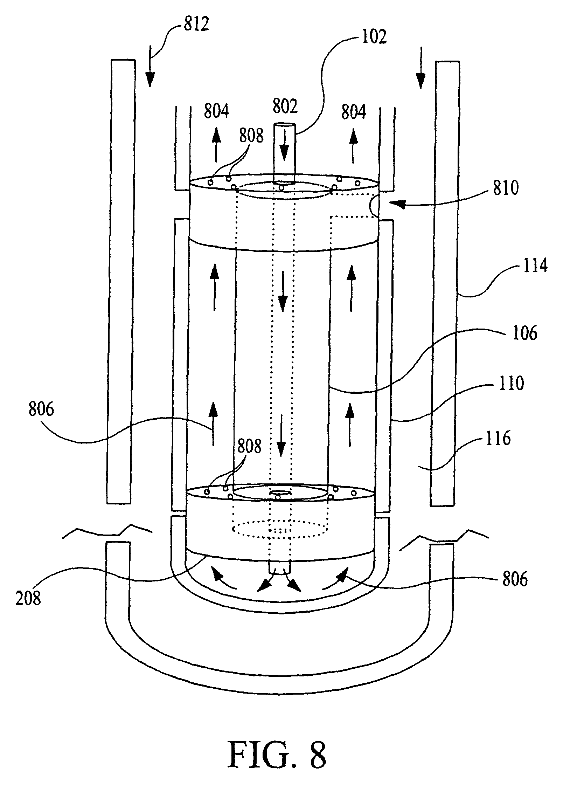

Figure 8 discloses a coaxial bioreactor with inlet port **802** and outlet ports **804** on one side of the bioreactor. In a preferred embodiment, this bioreactor is axially less than 40 millimeters long and the fibers are secured with manifolds or endcaps that have coaxial counter-sunk holes that fit the fiber diameters within ±5 mils of an inch. Optionally, hepatocytes center the inner fiber, thereby making construction easier and permitting axial scale-up. The bioreactor can be partially filled depending on inoculation procedure. The bioreactor is assembled with an inner fiber **102**, middle fiber **106**, outer fiber **110,** and manifolds **208** in a fashion similar to the two-sided multi-coaxial hollow fiber bioreactor, except there is only one inlet and one outlet. The one sided multi-coaxial bioreactor of Figure 8 uses the same fibers and NMR tube described above for the two-sided multi-coaxial hollow fiber bioreactor. In this embodiment housing **114** is an NMR tube. The specific design can be inserted in an 10 millimeter NMR probe used with conventional vertical bore magnets. Flow direction **806** can be switched by swapping inlet port **802** and outlet ports **804.** The flow rate in the outer annulus is partly controlled by the diameter and number of holes **808** aligned as a circle around the three manifolds. The manifolds and fibers are assembled as described above for the two-sided single multi-coaxial hollow fiber bioreactor. Also shown is inoculation port **810** and air flow **812**.

An additional one-sided multi-coaxial hollow fiber bioreactor design can be constructed by dead-ending the inner fiber **102** and omitting the holes **808** in the bottom manifold **812.** This changes the flow configuration by forcing the flow path from inner compartment **104** through the first middle compartment **108** where cells reside and exiting through the second middle compartment **112,** or vice versa. Since oxygenation occurs in the outer compartment in the instant invention, it is preferred to flow from second middle compartment **112** toward the inner compartment **104** so that media becomes appropriately oxygenated before entering the cell mass.

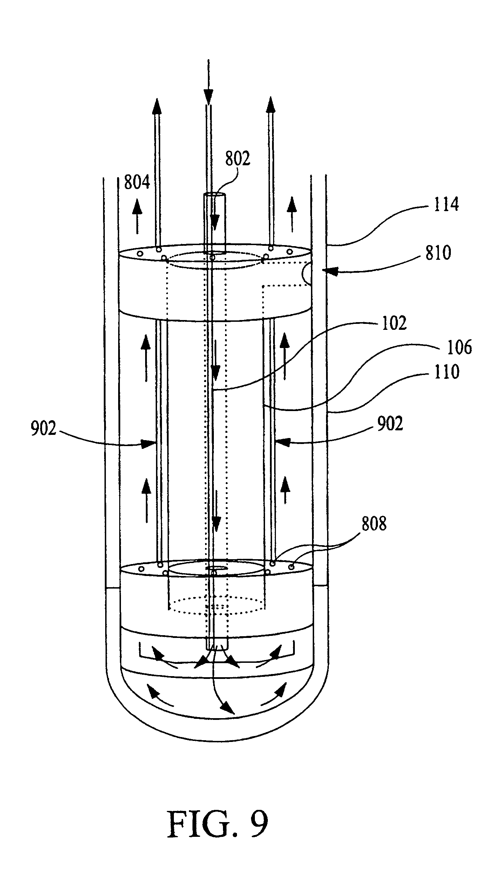

To enhance NMR sensitivity the one-sided bioreactor can be made to fit inside a smaller diameter NMR tube such as 8 to 15 mm. In order to achieve a smaller diameter, in one embodiment of the instant invention a 200 µm to 500 µm o.d. microbore aeration fiber serves as inner fiber **102,** a 1 to 1.3 millimeter o.d., middle fiber **106,** and a 3 to 4 millimeter o.d. outer fiber **110.** With oxygenation occurring by way of the inner compartment **104** the preferred flow is from the first middle compartment **108,** through cell mass in the second middle compartment **112,** and exiting by way of the outer compartment **116.** The gas chamber, of inner compartment **104,** is dead-ended, and gas is flushed from the compartments by way of diffusion to a flowing air stream connected to the gas chamber by way of a tee connectors at the top of the bioreactor. In order to have air circulate through the bioreactor, an air flow-through the design is needed. The inner fiber **102** in Figure 9 is manifolded at the bottom of the bioreactor and at least one 200 μm to 500 μm return microbore aeration fiber **902** is epoxied into holes **808** in the manifolds bordering the outer compartment **116** and used for media return. Alternatively, a two-fiber coaxial bioreactor is constructed from an inner fiber mini-oxygenator composed of 0.8 mm polypropylene fibers is placed in-line to the one-sided bioreactor and gas is heated by water-jackets to avoid outgasing due to temperature changes and/or differences between oxygenation device and bioreactor.

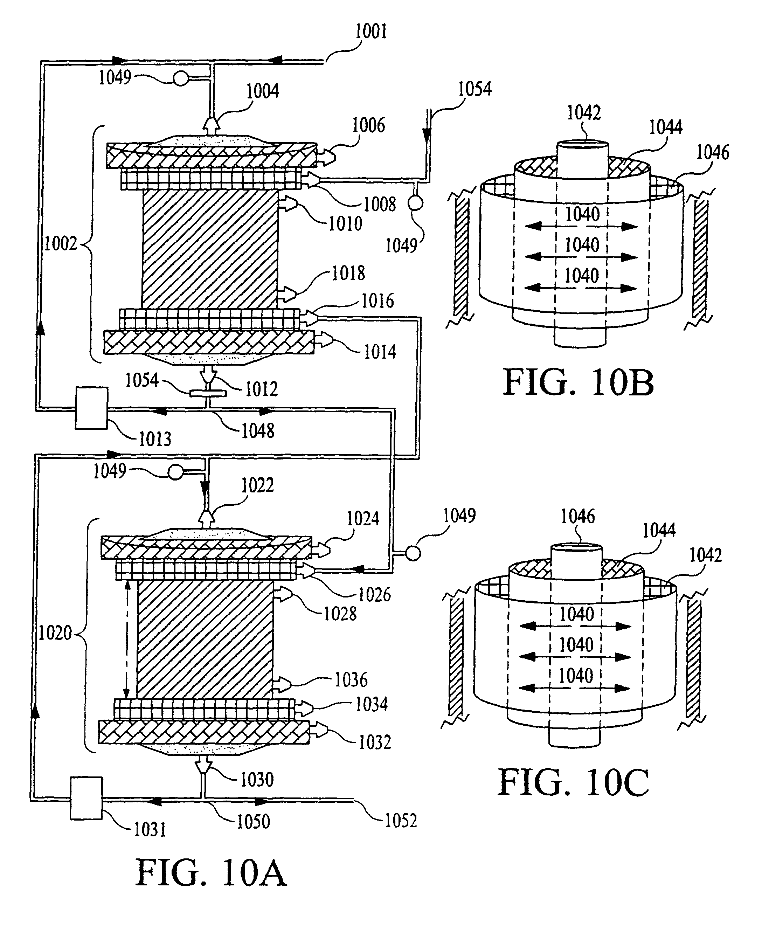

Figure 10A illustrates the serially-linked bioartificial liver. It includes at least two bioartificial liver subunits with potentially up to about 160 g of tissue per subunit. Cell suspensions are introduced into the respective annular compartments by way of ports **1006** and/or **1014** and **1024** and/or **1032**, and allowed to anchor to the annular compartments. Radial flow dynamics are used to perfuse media from plasma to cells for biotransformatory functions. The flow scheme is switched in the second subunit so synthetic factors can flow from the cells back into the plasma. Radial flow is discussed further, infra.

As shown in Figure 10A, plasma **1001** from the patient enters first bioartificial liver subunit **1002** through first plasma entrance port **1004,** media enters **1054** first bioartificial liver subunit **1002** through first media entrance port **1008** and gas enters first bioartificial liver subunit **1002** through first gas entrance port **1010**. Radial flow is produced by selecting hollow fibers with characteristics of pore size and pore number such that the hydraulic permeability of the fiber is relatively high and by having a substantial pressure gradient across the fibers as determined in the hydrodynamic model discussed below. Figure 10B depicts plasma flow under conditions where plasma compartment **1042** has higher pressure than cell compartment **1044** and media compartment **1046**. Under these conditions, some plasma flows radially from entrance port **1004** to plasma compartment **1042,** through cell mass contained in cell compartment **1044,** into media compartment **1046**. and out the media exit port **1016**. The remaining plasma then exits first bioartificial liver subunit **1002** through first plasma exit port **1012,** and the plasma is divided by tee-junction **1048**. Media exits first bioartificial liver subunit **1002** through first media exit port **1016** and gas exits first bioartificial liver subunit **1002** through first gas exit port **1018**. The elevated pressure in plasma compartment **1042** necessary to create the ΔP to drive radial flow is created by dividing the plasma stream at tee junction **1048** and rapidly recirculating plasma through plasma compartment **1042** by way of recirculating pump **1013** connecting the plasma entrance port **1004** to plasma exit port **1012**. The flow rates or velocities create sufficient head pressure at the plasma entrance port **1004** and pump rates are adjusted for desired ΔP which is measured by pressure gauges **1048** at ports **1004** and **1008**. The desired ΔP can also be created by insertion of a needle valve **1054** in the line between the plasma exit port **1012** and the tee junction **1048**. In this manner, plasma flow rate can remain constant through the first bioartificial liver subunit **1002** and the desired ΔP is achieved by modulating flow through the needle valve. The same principle can used to control radial flow in the second bioartificial liver subunit **1020**. In an alternative embodiment, plasma compartment **1042** is dead-ended by eliminating plasma exit port **1012**.

The flow scheme is then switched in the second subunit such that the fluid from exit port **1016** enters second bioartificial liver subunit **1020** through second media entrance port **1022,** plasma enters second bioartificial liver subunit **1020** through second plasma entrance port **1026** and gas enters second bioartificial liver subunit **1020** through second gas entrance port **1028**. A portion of the media flows radially from the entrance port **1022** through the annular cell layer and into the plasma component. The remaining media then exits second bioartificial liver subunit **1020** through second media exit port **1030** and the fluid stream is split at tee junction **1050** and a portion is conducted to means for returning it to the patient **1052**. Cells optionally exit second bioartificial liver subunit **1020** through second cell exit port **1032,** plasma exits second bioartificial liver subunit **1020** through second plasma exit port **1034** and gas exits second bioartificial liver subunit **1002** through second gas exit port **1036**. Figure 10C depicts plasma-containing media flow where the media compartment **1046** is now switched with the plasma compartment **1042**. As described above, the ΔP is established in the media compartment **1046** by rapid recirculation of the plasma-containing media by pump **1031** to create sufficient head pressure. The ΔP is measured by pressure gauges **1049** at ports **1022** and **1026**. In an alternative embodiment, sufficient head pressure is created by eliminating or restricting (using a needle valve or other similar device) the flow through the second media exit port **1030**.

In order to create radial flow, several flow configurations are possible. Flow rate differences in two compartments results in a pressure difference (ΔP) creating radial flow across the annular space, which is governed by Darcy's law:

$$v_r = k \ \Delta P/\eta \ L \hspace{5cm} (1)$$

where $v_r$ is the radial flow velocity, $k$ is the hydraulic permeability, a constant dependent on the physical features of the hollow fiber (including pore size and pore number), the solvent, and the solute, $\eta$ is the viscosity of the solution, and L is the length of the contact between the two compartments, here, essentially the fiber length. Any of a variety of pore sizes can be selected, including, but not limited to $10^{-6}$ m, $0.1 \times 10^{-6}$ in, and $0.05 \times 10^{-6}$ m. Thus radial flow can be enhanced by a suitable choice of fibers with a high hydraulic permeability and by modulation of flow rates.

A model based on Darcy's law permits one to estimate the correlation between pressure difference and radial flow given the hydraulic permeabilities of the inner fiber **102** and middle fiber **106.** The model assumes incompressible and Newtonian fluid, and that the axial pressure gradient is negligible, and the flow rate across the fibers was constant. Deriving this equation for two concentric hollow fibers the following relationship is obtained.

$$\Delta P = \frac{Q}{2\pi L}\left[\frac{\ln\left(\frac{r_b}{r_a}\right)}{K_1} - \frac{\ln\left(\frac{r_d}{r_c}\right)}{K_2}\right] \hspace{3cm} (\text{II})$$

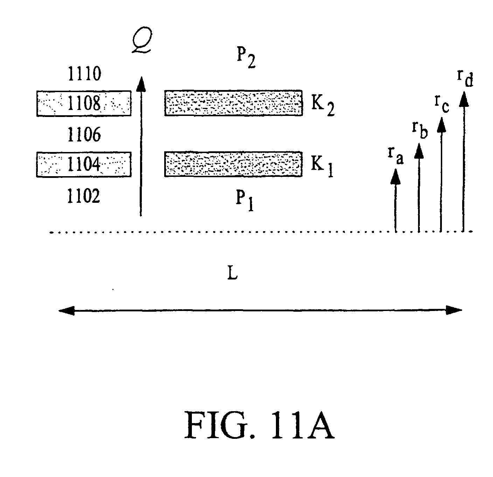

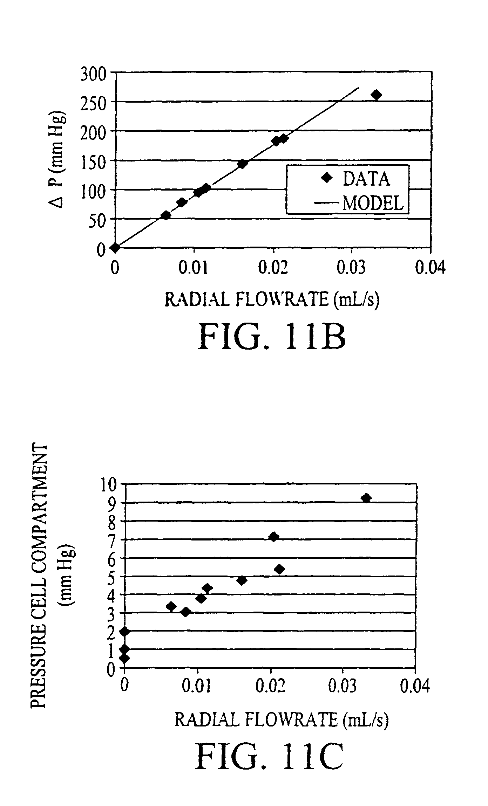

Figure 11A defines the variables used in the equation. Q is radial flow rate from compartment **1102** characterized by a hydrostatic pressure $P_1$, through pores in fiber **1104** characterized by hydraulic permeability $K_1$, through intermediate compartment **1106,** then through pores in second fiber **1108** characterized by hydraulic permeability $K_2$ to compartment **1110** characterized by hydrostatic pressure $P_2$. The radial distances from a centerline to the inside of fiber **1104** is $r_a$, to the outside fiber **1104** is $r_b$, to the inside fiber **1108** is $r_e$ and to the outside of fiber **1108** is $r_d$. It is to be understood that the direction of radial flow of any particular chemical constituent, including but not limited to oxygen, culture medium, plasma, and biosynthetic products, depends on the direction of the pressure differential. The directions of flow and of the corresponding radial flow rate can be either positive or negative, and are represented by the sign of the flow and of Q. The invention contemplates values of Q corresponding to flow from inner compartments to outer compartments, and equally contemplates values of Q corresponding to flow from outer compartments to inner compartments. Figure 11B compares the experimental data of radial flow from the second middle compartment **112** to the inner compartment **104** as a function of pressure difference to the modeled data using experimentally determined values of $K_1$ and $K_2$. Figure 11C illustrates the pressure in the cell compartment **1106** corresponding to the experiment shown in Figure 11B. Comparison of figure 11B to 11C shows that the majority of transmembrane pressure difference occurs across the middle fiber **106** and this is because of the relatively large hydraulic permeability and wall thickness of the middle fiber **106.** Therefore, cells are protected from the adverse effects of high pressure created in the second middle compartment **112,** and as the hydraulic permeability of the middle fiber **106** increases, the pressure is more easily transmitted to the first middle compartment **108** and cells becomes less protected from the pressure in the second middle compartment **112.** Therefore, this model can be generally used in the operation of the bioreactor to predict the appropriate pressure difference for given inner **102** and middle **106** fibers hydraulic permeabilities. In preferred embodiment, the device includes a software program based on this predictive model which aids in the selection of culture conditions regarding radial flow velocities and hydrostatic pressures in the cell compartment.

A method of selecting a radial flow rate in a bioreactor comprising semi-permeable fibers to enhance cell viability comprising: measuring a first hydraulic pressure associated with a first semi-permeable fiber and a second hydraulic pressure associated with a second semi-permeable fiber to obtain a pressure differential and adjusting the first hydraulic pressure, the second hydraulic pressure, or a combination thereof to select one or more radial flow rates so as to improve cell viability.

Thus, in one embodiment, the invention comprises a method of selecting a radial flow rate in a bioreactor comprising semi-permeable fibers to enhance cell viability comprising: measuring a first hydraulic pressure associated with a first semi-permeable fiber and a second hydraulic pressure associated with a second semi-permeable fiber to obtain a pressure differential and adjusting the first hydraulic pressure, the second hydraulic pressure, or a combination thereof to select one or more radial flow rates so as to improve cell viability. The first fiber and the second fiber can be coaxial. In a particular embodiment, the method of selecting a radial flow rate comprises selecting the radial flow rate based on the formula:

$$\Delta P = \frac{Q}{2\pi L}\left[\frac{\ln\left(\dfrac{r_b}{r_a}\right)}{K_1} - \frac{\ln\left(\dfrac{r_d}{r_c}\right)}{K_2}\right]$$

where $\Delta P$ is the pressure differential, Q is the radial flow rate, L is the length of the shorter of the first and second fiber lengths, $r_a$ is the radial distance from the centerline of the bioreactor to the inner surface of the first fiber, $r_b$ is the radial distance from the centerline of the bioreactor to the outer surface of the first fiber, $r_c$ is the radial distance from the centerline of the bioreactor to the inner surface of the second fiber, $r_d$ is the radial distance from the centerline of the bioreactor to the outer surface of the second fiber, $K_1$ is the hydraulic permeability of the first fiber, and $K_2$ is the hydraulic permeability of the second fiber.

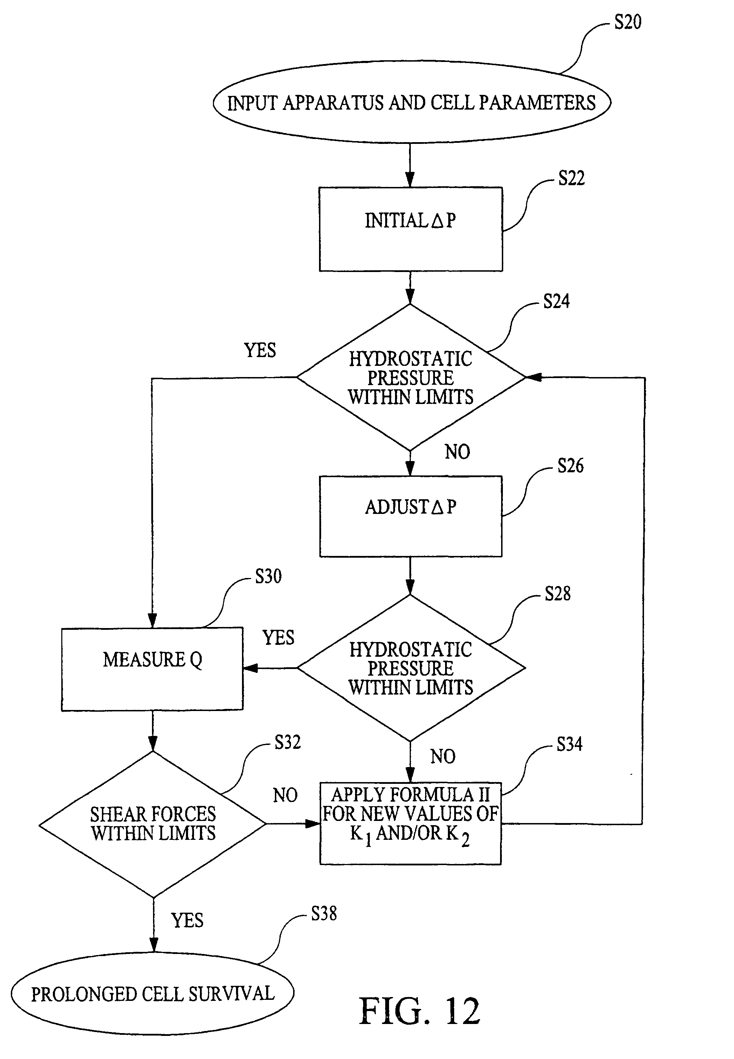

The software can be in the form of an algorithm as illustrated in Figure 12, and used to select mass transfer conditions for specific cell types. Initial $\Delta P$ values are obtained from a knowledge of characteristics of the bioreactor, including diffusion distance **118** and fiber length, as well as known physiological conditions in the tissue of interest.

A detailed description of the radial flow algorithm is provided in Figure 12. The first step shown in block **S20** begins with input of parameters of the bioreactor, including the geometry and dimensions of the bioreactor, and parameters of the cells, including resistance to hydrostatic pressures and shear stress. The pressure differential is then sampled, block **S22.** The next step requires a determination of whether the pressure differential is within the limits of hydrostatic pressure consistent with continued viability of the cells used in the system, as shown in block **S24.** If the pressure differential is not within permissible limits, the pressure differential is adjusted, as shown in block **S26.** Then the hydrostatic pressure is again measured to determine if it is within permissible limits, as shown in block **S28.**

If the determination from either block **S24** or block **S28** is that the hydrostatic pressure is compatible with cell viability, then the radial flow is measured, as shown in block **S30.**

The next step requires a determination of whether the shear forces associated with radial flow are within viability limits for the cell type of interest, as shown in block **S32**. If the shear forces are not within permissible limits, the formula II, supra, relating $\Delta P$ and the radial flow rate Q is applied, as illustrated in block **S34.** As a result of the calculation of block **S34,** more suitable hydraulic permeability $K_1$ and/or hydraulic permeability $K_2$ is determined, as necessary **S36.**

Upon change in one or more hydraulic permeabilities, as in block **S34,** the system returns to block **S24** for further evaluation of first hydrostatic pressure and then shear force.

When the hydrostatic pressure is within limits consistent with cell viability, as in either block **S24** or block **S28,** then the radial flow rate, Q, is measured as in block **S30.** If the shear forces associated with the radial flow is consistent with cell viability, then conditions for cell survival are established, as illustrated in block **S38.** By setting suitably narrow limits for permissible hydrostatic pressure and shear force, an optimum condition for prolonged cell survival is attained.

In another embodiment, the invention can comprise a computer readable medium including instructions therein for calculating a radial flow rate in a bioreactor comprising semi-permeable fibers, said instructions including the steps of: (a) receiving measurements of hydraulic permeability for each of at least two semi-permeable fibers, (b) receiving hydraulic pressure measurements associated with a pressure differential for at least two coaxial semi-permeable fibers, and (c) estimating said radial flow rate between said coaxial semi-permeable fibers.

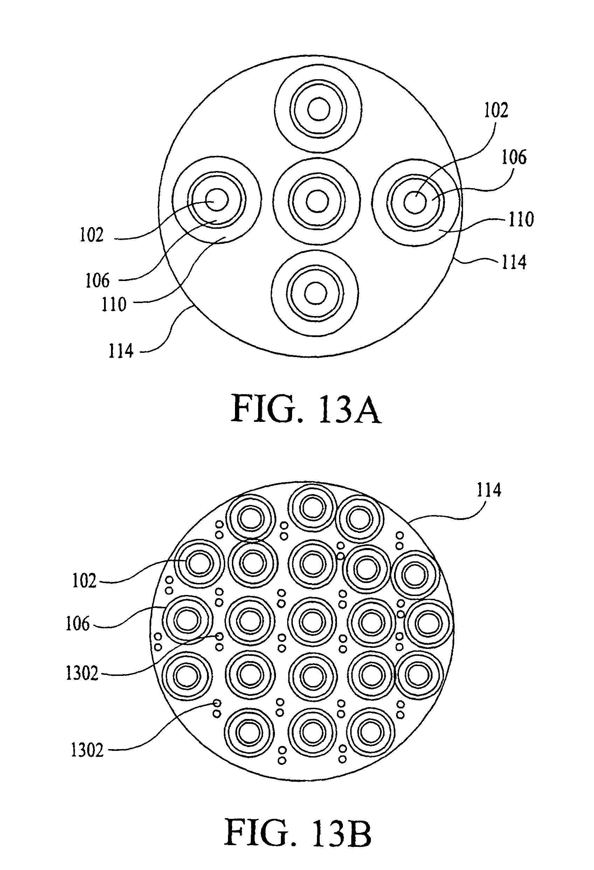

Figure 13A illustrates a cross section of a multi-coaxial bioreactor comprising three coaxial hollow fibers nested within each other. Figure 13A depicts innermost fiber **102,** middle fiber **106,** outer fiber **110** and housing **114.**

Figure 13B illustrates a cross section of an alternative embodiment of a multi-coaxial bioreactor. Figure 13B depicts innermost fiber **102** middle fiber **106,** smaller aeration fiber **1302** and housing **114.** Smaller standard aeration fiber **1302** is placed in the spaces formed by stacking coaxial fibers which comprise innermost fiber **102** and middle fiber **106.** Thus, a first compartment is formed by the inner wall of innermost fiber **102.** A second compartment is defined by the outer wall of innermost fiber **102** and the inner wall of middle fiber **106.** A third compartment is defined by the outer wall of middle fiber **106** and housing **114.** A fourth compartment is formed by the inner wall of smaller aeration fibers **1302.** In alternative embodiments, a fifth compartment can be created for aeration purposes by inserting a fourth, small aeration fiber inside innermost fiber **102.**

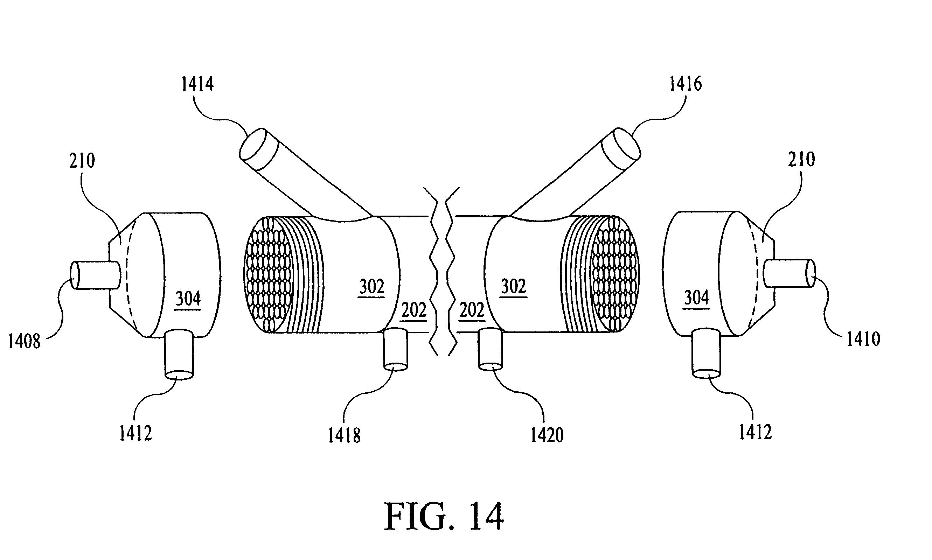

Figure 14 illustrates an embodiment of a bioreactor. The following components are shown: first manifold **302,** second manifold **304,** endcap **210,** inner compartment inlet **1408,** inner compartment outlet **1410,** cell compartment **1412,** gas inlet **1414,** gas outlet **1416,** outermost compartment inlet **1418,** outermost compartment outlet **1420** and housing **202.**

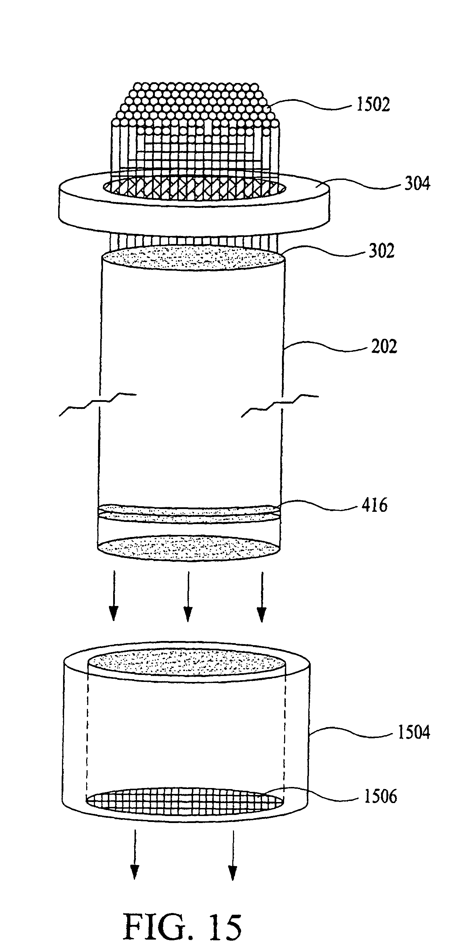

Figure 15 illustrates a device used in the construction of a bioreactor. Components shown include plurality of fibers **1502,** second manifold **304,** first manifold **302,** housing **202,** O-ring **416,** vacuum head **1504,** and mesh **1506.** The vacuum allows for rapid insertion of smaller fibers into larger fibers. Mesh **1506** retains the fibers in place.

In scaled-up versions of the multi-coaxial bioreactor, manifolds consist of multiple coaxial holes for the various fibers, and dowels are placed in holes precisely located on the manifolds to insure that all multi-fiber units are coaxial. Since multi-fiber units are reproduced accurately due to the coaxial design, experimental parameters from a single multi-fiber unit are directly applied to the scaled-up bioreactor.

The construction of the bioreactor may have many variations that will be evident to the practitioner. As one example, the hollow fibers may vary in pore size, length, wall thickness and composition and the hydrodynamic model shown above and in Figure 11 can be used to determine the optimum hollow fiber characteristics. The hollow fibers may be made of any of a number of materials including, but by no means limited to polysulfone, cellulose acetate, mixed esters of cellulose, regenerated cellulose, polyvinylalcohol, polyurethane, polyvinylidine, polypropylene, polycarbonate, and polyamide. Cellulose acetate is more permeable to nutrient media and polypropylene is more permeable to gases and those skilled in the art will select appropriate fibers based on these and other properties. The means of formation of pores and control of the pore size will be obvious to one of skill in the art and may include, but in no way be limited to, neutron bombardment, controlled polymerization, and incorporation of leachable agents. Any of several adhesives including polyurethane epoxy may be used for potting the fibers, and those skilled in the art will know of appropriate adhesives.

## 6. Examples

The following specific examples are provided to better assist the reader in the various aspects of practicing the present invention. As these specific examples are merely illustrative, nothing in the following descriptions should be construed as limiting the invention in any way. Such limitations are, or course, defined solely by the accompanying claims.

### 6.1 NMR analysis of liver cell function in the one-sided multi-coaxial hollow fiber bioreactor.

Sprague-Dawley rats are anesthetized with pentobarbital (50 mg/kg intraperitoneally). The liver is exposed by a ventral midline incision and the portal vein is cannulated for infusion of cell dissociation solutions. The liver cells are dissociated by sequential infusions of ethylene diamine tetraacetic acid (50 mM) and collagenase (1 to 20 mg/ ml) in Krebs-Henseleit buffer, pH 7.4. Adequate perfusion of the liver is indicated by uniform blanching of the liver. Isolated cells are collected and introduced into the cell compartment (compartment 2) of the one-sided multi-coaxial hollow fiber bioreactor.

Nuclear magnetic resonance (NMR) is performed using an NMR probe design composed of two Helmholtz coils photo-etched onto flexible copper-coated composite. The two coils, suitably insulated, are wrapped around the bioreactor and oriented orthogonally to each other. The inner coil is tuned to 81 MHz for study of energy metabolism as measured by changes in the spectrum of $^{31}$P. The probe and bioreactor assembly is placed on a centering cradle in the isocenter of the magnet for optimal comparison of spectra. The aerated nutrient medium is supplied to the first compartment inlet port of the bioreactor. Integral aeration is provided by flow of a 95% air with 5% $CO_2$ mix through inlet port 4, associated with the outermost or fourth compartment of the bioreactor. Ham's F-12 nutrient medium is pumped through compartment 3 with a peristaltic pump. The temperature of the reservoir of medium is maintained at 42 °C with a temperature controlled water bath, so as to maintain the bioreactor temperature at 37 °C. The NMR signal from $\gamma$-$^{31}$P nucleotide triphosphates and $\beta$-$^{31}$P nucleotide diphosphates, other cellular components of energy metabolism, and biosynthesis are analyzed. The NMR signal is monitored as a function of mass transfer dictated by gas flow rate and oxygen percentage, nutrient medium flow rates, and cell loading densities.

### 6.2 Oxygen flux in the absence of cells.

Oxygen microelectrodes are connected to a transducer and Workbench™ software, and then calibrated against known standards. The calibrated oxygen microelectrodes are placed at intervals along the fiber length in compartment 2 of the multi coaxial hollow fiber bioreactor. A reservoir of plasma is attached to the inlet port of compartment 1, the innermost compartment of the multi-coaxial hollow fiber bioreactor. A reservoir of RPMI 1640 nutrient medium is attached to the inlet port of compartment 3. Peristaltic pumps are arranged in-line to circulate the plasma and nutrient medium. Compartment 2 is also filled with nutrient medium. The signal from each microelectrode is acquired at ten-second intervals and processed by the software for conversion to oxygen tensions. The gas phase is switched between 95% air with 5% $CO_2$ and 95% $N_2$ with 5% $CO_2$ at selected intervals. Rates of depletion and recovery of oxygen tension are measured at different flow rates to evaluate oxygen flux in the absence and presence of cells.

### 6.3 Use As An Extracorporeal Liver Assist Device for Evaluation of Bilirubin.

<u>Background.</u> The Gunn rat model, which is the animal model for Crigler-Naijar syndrome in humans is an ideal model for demonstrating the efficacy of the bioreactor as an extracorporeal liver assist device. The Gunn rat has a defect inherited as an autosomal recessive trait in Wistar rats. The defect, present in homozygous recessive animals, is in the gene encoding UDP-glucuronosyltransferase, an enzyme necessary for the conjugation and biliary excretion of bilirubin (a breakdown product of hemoglobin in senescent red blood cells). The Gunn rat therefore cannot conjugate and excrete bilirubin and becomes hyperbilirubinemic, having serum bilirubin levels of about 5-20 mg/dL, compared with 1 mg/dL in normal rats.

<u>Exprimental Protocol.</u> A scaled-up multi-coaxial hollow fiber bioreactor is used for the assembly of a device acting as an extracorporeal liver assist device with Gunn rats. The livers of heterozygous (phenotypically normal) Gunn rats are perfused and the cells are isolated. The cells are suspended in Dulbecco's Modified Eagle Medium (DMEM) and $10^9$ cells are introduced into the second compartment of the bioreactor. Blood from the femoral artery of a Gunn rat (total average blood volume ca. 10 to 12 mL) is perfused through compartment 3 of the bioreactor, separated from the liver cell annular space by the wall of the hollow fiber, at a flow rate of about 0.6-0.8 mL/min with the aid of a peristaltic pump. At the same time, DMEM is flowed through the compartment one of the bioreactor at a flow rate of about 0.5 mL/min. Blood flowing out of the bioreactor is returned to the Gunn rat.

The levels of unconjugated and conjugated bilirubin in blood exiting the bioreactor are determined over the course of six hours using the Sigma Total and Direct Bilirubin assay system according to the instruction supplied by Sigma Chemical Company (Sigma Procedure #522/553).

### 6.4 Biosynthetic hepatocyte function in a scaled-up multi-coaxial hollow fiber bioreactor/ BAL.

Liver cells isolated as in Example I are further separated by zonal centrifugation in sucrose density gradients. Density fractions corresponding to parenchymal cells are collected and introduced into the aseptic cell compartment (compartment 2) of the scaled-up multi-coaxial bioreactor.

The parenchymal cells are maintained by circulating warm Ham's F-12 nutrient medium through compartments 1 and 3, and 95% air with 5% $CO_2$ through compartment 4. The effluent from compartment 1 is collected and fractions are analyzed for parameters of biosynthetic liver function. Albumin synthesis is measured by enzyme-linked immunosorbent assay.

### 6.5 Biotransformatory function in a scaled-up multi-coaxial hollow fiber bioreactor/BAL.

Liver cells isolated as in Example I are further separated by zonal centrifugation in sucrose density gradients. Density fractions corresponding to Kupffer cells are collected and introduced into the compartment 2 (cell compartment) of the scaled-up multi-coaxial hollow fiber bioreactor.

The cells in the bioreactor are maintained by circulating DMEM (without Phenol Red) through the inlet and outlet ports for compartments I and 3 and 95% air with 5% $CO_2$ through the ports for compartment 4. The cells are permitted to adhere within the compartment, followed by the introduction of free hemoglobin (1-10 mg/ml) into compartment 1. The appearance of hemoglobin and the metabolic products of hemoglobin in compartment 3 are monitored with an in-line spectrophotometer.

### 6.6 The serially-linked bioreactor with human cells

Human hepatoma C3A cells are cultured as described (Mickelson, J.K. et al. *Hepatology* **1995,** 22, 866) and introduced into all the second compartments of the serially-linked bioreactor. Nutrient medium and 95% air with 5% $CO_2$ are pumped through the third and outermost compartments, respectively, and cell growth is monitored by glucose utilization. When the cells have attained the plateau, or stationary, growth phase, the albumin output is monitored.

The blood of a patient suffering liver failure is separated into plasma and cells by plasmapheresis and the plasma is pumped into the first compartment of the first bioartificial liver subunit. A portion of the plasma flows radially from the first compartment through the cell compartment to the third compartment to form biotransformed effluent. The plasma exits the first compartment of the first bioartificial liver subunit and flows into the third compartment ofthe second bioartificial liver subunit. The biotransformed effluent from the third compartment of the first bioartificial liver subunit flows into the first compartment of the second bioartificial subunit. Radial flow in the first bioartificial liver subunit detoxifies a portion of the plasma and radial flow in the second bioartificial liver subunit contributes biosynthetic products to the plasma to form supplemented plasma. Vital signs, jaundice, and blood level of toxins are monitored at regular intervals. Flow rates of plasma and medium are adjusted to maximize biotransformation of circulating toxins. Survival of the patient is measured.

*6.7 Extracellular matrix effects on differentiation of hepatocytes in the scaled-up multi-coaxial hollow fiber bioreactor.*

Parenchymal cells are isolated by zonal centrifugation as in example IV, above, suspended in reconstituted basement matrix from the Englebreth-Holm-Swarm mouse sarcoma, and introduced into the compartment 2 (cell compartment) of the scaled-up multi-coaxial bioreactor. The hepatocytes are arrested in a Go state by adhesion to the basement matrix, and are maintained in the normal hepatic phenotype (Rana et al., 1994). The highly differentiated state is characterized by synthesis of albumin and hepatic transcription factors such as C/EBP-. The parenchymal cells are maintained by circulating warm Ham's F-12 nutrient medium through compartments 1 and 3, and 95% air with 5% $CO_2$ through compartment 4. The effluent from compartment 1 is collected and fractions are analyzed for parameters of biosynthetic liver function. Albumin synthesis is measured by enzyme-linked immunosorbent assay.

*6.8 Growth and differentiation of human hepatocytes in the scaled-up multi-coaxial hollow fiber bioreactor.*

Human parenchymal hepatocytes are isolated by the method of (Block, G.D. et al. *J Cell Biol* **1996,** *132,* 1133) and introduced into compartment 2 of the scaled-up multi coaxial hollow fiber bioreactor. The parenchymal cells are propagated by exposure to hepatocyte growth factor (HGF/SF), epidermal factor, and transforming growth factor alpha in nutrient medium HGM introduced into compartment 3 and air:$CO_2$ (19:1) introduced into compartment 4. The ratio of transcription factor C/EBP to C/EBP is decreased by this process and the cell synthesis of albumin also is decreased. The medium flowing through compartment 3 is modified to include transforming growth factor and epidermal growth factor to induce differentiation of the cells and synthesis of albumin, in the formulation described (Sanchez, A. et al. *Exp Cell Res* **1998**, *242*, 27).

*6.9 Biosynthesis of hormones and factors in the scaled-up multi-coaxial hollow fiber bioreactor.*

Parathyroid glands are obtained aseptically, minced, and treated with collagenase as described (Hornicek, F.L. et al. *Bone Miner* **1988**, *4*, 157). The dispersed cells are suspended in CMRL-1415 nutrient medium supplemented with fetal bovine serum and introduced into compartment 2 of the scaled-up multi-coaxial bioreactor. A mixture of 95% air and with 5% $CO_2$ is pumped through port 4. Warm medium is pumped through ports 1 and 3 and the effluent from the chamber is concentrated by ultrafiltration for collection of parathyroid hormone, parathyroid hypertensive factor, and other cell products. The hormones and factors are purified by immunoprecipitation and chromatography.

*6.10 The five compartment serially-linked bioreactor with human cells*

Human hepatoma C3A cells are grown as in example VI, above, except in the third compartment of a five-compartment serially-linked bioreactor. The innermost compartment (compartment 1) and the outermost compartment (compartment 5) are suffused with the gas mix, 95% air with 5% $CO_2$. Nutrient medium is pumped through the second and fourth compartments, respectively, and cell growth is monitored by glucose utilization. When the cells have attained the plateau, or stationary, growth phase, the albumin output is monitored.

The blood of a patient suffering liver failure is separated into plasma and cells by plasmapheresis and the plasma is pumped through the serially connected second compartments of the bioreactor. Vital signs, jaundice, and blood level of toxins are monitored at regular intervals. Flow rates of plasma and medium are adjusted to maximize biotransformation of circulating toxins. Survival of the patient is measured.

*6.11 Construction of the five compartment serially-linked bioreactor with tight-packed fiber arrangement and use with human cells*

Bioreactor subunits are constructed with 300 outermost coaxial fibers (3 millimeter o.d.) forming a part of the outer boundary of the annular cell compartments. A hollow fiber of 1.3 millimeter o.d. is inserted into each of the 3 millimeter fibers, forming part of the inner boundary of the annular cell compartments and part of the outer boundary of the compartments number 2. One micro bore hollow fiber (300 μm o.d.) is inserted into each of the 1.3 millimeter fibers as the innermost compartment of each coaxial fiber module and another set of 300 microfibers is placed parallel and adjacent to the coaxial modules. Both sets of microfibers carry aeration gas to oxygenate the plasma and the medium. The outermost compartment is compartment number 4 and is formed by the outside of the outermost coaxial fibers (of 3 millimeter o.d.), the outside of the microfibers that are adjacent to the coaxial modules, and the inside of the housing.

The serially-linked bioreactor and associated tubing and connections are sterilized.

Human hepatocytes are isolated as in Example VIII and introduced into the annular cell compartments, compartments 3, of each of two bioreactor subunits. The hepatocyte cells are propagated by exposure to nutrient medium and growth factors described in Example VIII until the cell density is sufficient for treatment of a patient in liver failure. The bioreactor subunits with a complement of viable liver cells are now termed BAL subunits.

When the bioreactor is used for the assembly of a device for patient treatment, patient plasma enters compartment number 2 of the first BAL subunit, through the first plasma entrance port, media enters compartment number 4 of the first BAL through the first media entrance port and gas enters the first BAL through first gas entrance port. Radial flow in the first subunit is produced by a pressure gradient across the fibers, such that the hydraulic pressure in the plasma compartment is higher than the pressure in the cell or media compartments. In consequence, part of the plasma flows from compartment number 2 through the annular cell compartment into compartment number 4. In the process the liver cells in the cell compartment can biologically detoxify the plasma passing through the cell compartment. As the detoxified plasma flows into compartment 4 the medium is modified with a detoxified plasma component. The remaining plasma then exits the first BAL subunit through the first plasma exit port, media exits the first BAL subunit through the first compartment number 4 exit port and gas exits the first BAL subunit through the first gas exit port. The flow scheme is then switched in the second subunit such that the fluid (medium containing detoxified plasma) from the first compartment number 4 exit port enters the second BAL subunit through the second compartment number 2 entrance port. Similarly, plasma from first compartment 2 exit port enters the second BAL subunit through the second compartment 4 entrance port and gas enters the second BAL subunit through the second gas entrance port. A portion of the media flows radially from the compartment number 2 entrance port through the annular cell layer and into the second compartment number 4. The liver cells add proteins and other biosynthetic products to the medium that flows through the cell compartment in subunit 2. As this enriched medium flows into the remaining plasma, the resultant, modified plasma is in part detoxified and in part enriched with biosynthetic proteins. The remaining medium then exits the second BAL subunit through the second compartment 2 exit port, the modified plasma exits the second BAL subunit through the second compartment 4 exit port and gas exits the second BAL subunit through the second gas exit port. The modified plasma, effluent from second compartment 4, is conducted to means for returning it to the patient to sustain life.

### 6.12 *Manufacture of scaled up multi-coaxial hollow fiber bioreactor.*

An apparatus is used to assemble a bioreactor in which a vacuum head, that is attached as necessary to a negative pressure source, holds a bundle of hollow fibers against a mesh for rapid insertion of smaller fibers. The opposite end of the hollow fibers is inserted into a manifold and placed in a vessel. polyurethane epoxy is applied to bond the hollow fibers to the manifold and the assembly of manifold, epoxy, and hollow fibers subjected to a centrifugal force to remove epoxy from the inside of the fibers. Upon curing, the free ends of the hollow fibers are trimmed with a saw. The process is repeated for insertion of the next smaller set of hollow fibers by applying the vacuum, gluing the ends to the next manifold, centrifuging, and trimming.

### Claims

1. A bioreactor, comprising:

   (a) a housing having an inner side comprising: a gas introduction means integral to the housing: and a gas expiration means integral to the housing;
   (b) an array of a plurality of modules of hollow fibers, residing within the housing, each module comprising:

      (i) a plurality of coaxial hollow fibers, each having an inner side and an outer side, including an innermost hollow fiber and an outermost hollow fiber;
      (ii) a plurality of compartments, comprising: a first compartment defined by the inner side of the innermost hollow fiber; and
      (iii) at least one additional compartment defined by a respective annular space between adjacent fibers of the plurality of coaxial hollow fibers; and

   (c) an outermost compartment defined by a space within the inner side of the housing which is not occupied by the plurality of modules
   (d) where the first compartment, the at least one additional compartment and the outermost compartment each further comprise at least one inlet port; and at least one outlet port, and
   (e) where the housing further comprises:

(i) at least one inlet manifold and at least one outlet manifold for the first compartment; and

(ii) at least one inlet manifold and at least one outlet manifold for each additional compartment.

2. The bioreactor of claim 1, where the hollow fibers are semipermeable.

3. The bioreactor of claim 2, where the hollow fibers comprise a material selected from the group consisting of polysulfone, polypropylene, nylon, polyester, polytetrafluoroethylene, cellulose acetate, and mixed esters of cellulose.

4. The bioreactor of claim 1, where the bioreactor further comprises at least $10^9$ cells.

5. The bioreactor of claim 4, where the cells are liver cells.

6. The bioreactor of claim 5, where the liver cells are selected from the group consisting of porcine liver cells and human liver cells.

7. The bioreactor of claim 1, where the at least one manifold further comprises a flow distributor.

8. The bioreactor of claim 7, where at least one compartment further comprises a extracellular matrix.

9. The bioreactor of claim 1, where at least one annular space is about 0.2 millimeters to about 0.8 millimeters.

10. The bioreactor of claim 1, where the bioreactor is sterilized by a means selected from the group consisting of autoclaving, ethylene oxide and gamma radiation.

11. The bioreactor of claim 1, wherein the innermost hollow fiber has a length of about 2 centimeters to about 50 centimeters.

12. The bioreactor of claim 1, where the housing has a first end and a second end and where each inlet port and each exit port are at the first end of the housing.

13. The bioreactor of claim 1, further comprising: microfibers substantially parallel to the modules of hollow fibers.

14. The bioreactor of claim 13, where the microfibers further comprise at least one aeration inlet port and at least one aeration outlet port.

15. The bioreactor of claim 1, where at least one coaxial hollow fiber is saturated with perfluorocarbon.

16. The bioreactor of claim 1, where at least one coaxial hollow fiber has a pore size of less than $1 \times 10^{-6}$m.

17. The bioreactor of claim 1, where at least one coaxial hollow fiber has a pore size of less than $0.1 \times 10^{-6}$m.

18. The bioreactor of claim 1, where at least one coaxial hollow fiber has a pore size less than $0.05 \times 10^{-6}$ m.

19. The bioreactor of claim 1, where at least one compartment further comprises cells mixed with an extracellular matrix.

20. Use of the bioreactor of claim 1 for the assembly of a device for supplying cell biosynthesis products to a patient in need thereof and comprising: means for pumping intravenous feeding solution through a compartment of said bioreactor; means for collecting the output, and means for intravenously feeding said output to the patient.

21. A serially-linked bioreactor, comprising a plurality of bioreactor subunits, each bioreactor subunit comprising:

(a) a housing having an inner side comprising: a gas introduction means integral to the housing; and a gas expiration means integral to the housing;

(b) an array of a plurality of modules of hollow fibers, residing within the housing, each module comprising:

(i) a plurality of coaxial hollow fibers, each having an inner side and an outer side, including an innermost hollow fiber and an outermost hollow fiber;

(ii) a plurality of compartments, comprising: a first compartment defined by the inner side of the innermost hollow fiber; and at least one additional compartment defined by a respective annular space between adjacent fibers of the plurality of coaxial hollow fibers; and

(c) an outermost compartment defined by a space within the inner side of the housing which is not occupied by the plurality of modules; and

(d), where the first compartment, the at least one additional compartment and the outermost compartment each further comprise at least one inlet port and at least one outlet port,

(e) where the housing further comprises;

(i) at least one inlet manifold and at least one outlet manifold for the first compartment, and

(ii) at least one inlet manifold and at least one outlet manifold for each additional compartment, and

(f) at least one compartment of one bioreactor subunit linked serially to at least one compartment of at least one other bioreactor subunit

22. The bioreactor of claim 21, where each bioreactor subunit further comprises at least $10^9$ cells.

23. The bioreactor of claim 22, where the cells are liver cells.

24. The bioreactor of claim 23 where the cells are selected from the group consisting of human liver cells and porcine liver cells.

25. The bioreactor of claim 22, where at least one compartment of each bioreactor subunit further comprises an extracellular matrix.

26. Use of the serially linked bioreactor of claim 21 for the assembly of a device for providing supplemented plasma products to a patient in need thereof, comprising

(a) means for introducing plasma of a patient into a bioreactor subunit of the serially linked bioreactor of claim 21,

(b) means for forcing at least a portion of the plasma to flow radially through a cell compartment of the bioreactor subunit to form a biotransformed effluent;

(c) means for introducing the biotransformed effluent into a second bioreactor subunit of the bioreactor of claim 21;

(d) means for forcing at least a portion of the biotransformed effluent to flow radially through a cell compartment of the second bioreactor subunit to form supplemented plasma; and

(e) means for returning the supplemented plasma to the patient's circulatory system.

27. A multi-coaxial hollow fiber bioreactor, comprising:

(a) a housing comprising an inner side; and

(b) a module of hollow fibers, comprising: at least three coaxial semipermeable hollow fibers, including an innermost fiber having an inner side, the inner side defining a first compartment which comprises at least one innermost inlet port and at least one innermost outlet port; a plurality of compartments, each compartment defined by a respective annular space between adjacent fibers of the at least three hollow fibers, including at least one outer inlet port and at least one outer outlet port,

where each compartment comprises a flow communication means between the respective annular space, the respective outer inlet port and the respective outer outlet port and where one of the annular spaces contains eucaryotic cells; and

(c) an outermost compartment defined by a space between the outer side of the outermost fiber of said at least three hollow fibers, and the inner side of the housing, and comprising at least one outermost inlet port, and at least one outermost outlet port;

(d) the housing comprising at least one inlet manifold and at least one outlet manifold for each of the compartments; and

(e) where at least one of the compartments contains eucaryotic cells.

28. Use of the bioreactor of claim 27 for the assembly of a device for cell culture comprising:

means for introducing viable cells into a compartment of said bioreactor, and
means for passing nutrient medium through coaxially adjacent hollow fibers.

**Patentansprüche**

1. Bioreaktor, umfassend:

   (a) ein Gehäuse mit einer Innenseite, umfassend: ein in dem Gehäuse integrales Mittel zur Gaseinführung und ein in dem Gehäuse integrales Mittel zur Gasausströmung,
   (b) eine Anordnung einer Vielzahl Hohlfasermodule, die sich in dem Gehäuse befindet, wobei jedes Modul umfasst:

   (i) eine Vielzahl koaxialer Hohlfasern, wobei jede eine Innenseite und eine Außenseite besitzt, einschließlich einer innersten Hohlfaser und einer äußersten Hohlfaser,
   (ii) eine Vielzahl Kompartimente, umfassend: ein erstes Kompartiment, das durch die Innenseite der innersten Hohlfaser definiert ist, und
   (iii) mindestens ein zusätzliches Kompartiment, das durch einen jeweiligen annularen Zwischenabstand zwischen benachbarten Fasern der Vielzahl koaxialer Hohlfasern definiert ist, und

   (c) ein äußerstes Kompartiment, das durch einen Zwischenraum innerhalb der Innenseite des Gehäuses definiert ist, welcher nicht durch die Vielzahl Module eingenommen wird, und
   (d) wobei das erste Kompartiment, das mindestens eine zusätzliche Kompartiment und das äußerste Kompartiment jedes weiterhin mindestens eine Einlassöffnung und mindestens eine Auslassöffnung umfassen, und
   (e) wobei das Gehäuse weiterhin umfasst:

   (i) mindestens einen Einlassverteiler und mindestens einen Auslassverteiler für das erste Kompartiment, und
   (ii) mindestens einen Einlassverteiler und mindestens einen Auslassverteiler für jedes zusätzliche Kompartiment.

2. Bioreaktor nach Anspruch 1, worin die Hohlfasern semipermeabel sind.

3. Bioreaktor nach Anspruch 2, worin die Hohlfasern ein Material umfassen, das ausgewählt ist aus Polysulfon, Polypropylen, Nylon, Polyester, Polytetrafluorethylen, Zelluloseacetat und den gemischten Estern von Zellulose.

4. Bioreaktor nach Anspruch 1, wobei der Bioreaktor weiterhin mindestens $10^9$ Zellen umfasst.

5. Bioreaktor nach Anspruch 4, worin die Zellen Leberzellen sind.

6. Bioreaktor nach Anspruch 5, worin die Leberzellen ausgewählt sind aus Schweineleberzellen und menschlichen Leberzellen.

7. Bioreaktor nach Anspruch 1, worin der mindestens eine Verteiler weiterhin einen Strömungsverteiler umfasst.

8. Bioreaktor nach Anspruch 7, worin mindestens ein Kompartiment weiterhin eine extrazelluläre Matrix umfasst.

9. Bioreaktor nach Anspruch 1, worin mindestens ein annularer Zwischenabstand etwa 0,2 mm bis etwa 0,8 mm beträgt.

10. Bioreaktor nach Anspruch 1, wobei der Bioreaktor sterilisiert ist mit einem Mittel, das ausgewählt ist aus einem Autoklaven, Ethylenoxid und Gammastrahlung.

11. Bioreaktor nach Anspruch 1, worin die innerste Hohlfaser eine Länge von etwa 2 cm bis etwa 50 cm besitzt.

12. Bioreaktor nach Anspruch 1, worin das Gehäuse ein erstes Ende und ein zweites Ende besitzt und worin jede Einlassöffnung und jede Auslassöffnung sich an dem ersten Ende des Gehäuses befinden.

**13.** Bioreaktor nach Anspruch 1, weiterhin umfassend: Mikrofasern, die im wesentlichen parallel zu den Hohlfasermodulen sind.

**14.** Bioreaktor nach Anspruch 13, worin die Mikrofasern weiterhin mindestens eine Belüftungseinlassöffnung und mindestens eine Belüftungsauslassöffnung umfassen.

**15.** Bioreaktor nach Anspruch 1, worin mindestens eine koaxiale Hohlfaser mit Perfluorkohlenstoff getränkt ist.

**16.** Bioreaktor nach Anspruch 1, worin mindestens eine koaxiale Hohlfaser eine Porengröße von weniger als $1 \times 10^{-6}$ m besitzt.

**17.** Bioreaktor nach Anspruch 1, worin mindestens eine koaxiale Hohlfaser eine Porengröße von weniger als $0,1 \times 10^{-6}$ m besitzt.

**18.** Bioreaktor nach Anspruch 1, worin mindestens eine koaxiale Hohlfaser eine Porengröße von weniger als $0,05 \times 10^{-6}$ m besitzt.

**19.** Bioreaktor nach Anspruch 1, worin mindestens ein Kompartiment weiterhin Zellen umfasst, die mit einer extrazellulären Matrix gemischt sind.

**20.** Verwendung des Bioreaktors nach Anspruch 1 zum Aufbau einer Vorrichtung zur Zuführung von Zellbiosyntheseprodukten an einen Patienten, der diese benötigt, und welche umfasst: Mittel zum Pumpen intravenöser Zufuhrlösung durch ein Kompartiment des Bioreaktors, Mittel zum Sammeln des Ausstoßes und Mittel zur intravenösen Zuführung des Ausstoßes an den Patienten.

**21.** Seriell verknüpfter Bioreaktor, umfassend eine Vielzahl von Bioreaktoruntereinheiten, wobei jede Bioreaktoruntereinheit umfasst:

(a) ein Gehäuse mit einer Innenseite, umfassend: ein in dem Gehäuse integrales Mittel zur Gaseinführung und ein in dem Gehäuse integrales Mittel zur Gasausströmung,
(b) eine Anordnung einer Vielzahl Hohlfasermodule, die sich in dem Gehäuse befindet, wobei jedes Modul umfasst:

(i) eine Vielzahl koaxialer Hohlfasern, wobei jede Faser eine Innenseite und eine Außenseite besitzt, einschließlich einer innersten Hohlfaser und einer äußersten Hohlfaser,
(ii) eine Vielzahl Kompartimente, umfassend: ein erstes Kompartiment, das durch die Innenseite der innersten Hohlfaser definiert ist, und mindestens ein zusätzliches Kompartiment, das durch einen jeweiligen annularen Zwischenabstand zwischen benachbarten Fasern der Vielzahl koaxialer Hohlfasern definiert ist, und

(c) ein äußerstes Kompartiment, das durch einen Zwischenraum innerhalb der Innenseite des Gehäuses definiert ist, welcher nicht durch die Vielzahl Module eingenommen wird, und
(d) wobei das erste Kompartiment, das mindestens eine zusätzliche Kompartiment und das äußerste Kompartiment jedes weiterhin mindestens eine Einlassöffnung und mindestens eine Auslassöffnung umfassen, und
(e) wobei das Gehäuse weiterhin umfasst:

(i) mindestens einen Einlassverteiler und mindestens einen Auslassverteiler für das erste Kompartiment, und
(ii) mindestens einen Einlassverteiler und mindestens einen Auslassverteiler für jedes zusätzliche Kompartiment, und

(f) mindestens ein Kompartiment von einer Bioreaktoruntereinheit, das seriell mit mindestens einem Kompartiment von mindestens einer anderen Bioreaktoruntereinheit verknüpft ist.

**22.** Bioreaktor nach Anspruch 21, worin jede Bioreaktoruntereinheit weiterhin mindestens $10^9$ Zellen umfasst.

**23.** Bioreaktor nach Anspruch 22, worin die Zellen Leberzellen sind.

**24.** Bioreaktor nach Anspruch 23, worin die Zellen ausgewählt sind aus menschlichen Leberzellen und Schweineleberzellen.

**25.** Bioreaktor nach Anspruch 22, worin mindestens ein Kompartiment von jeder Bioreaktoruntereinheit weiterhin eine extrazelluläre Matrix umfasst.

**26.** Verwendung des seriell verknüpften Bioreaktors aus Anspruch 21 zum Aufbau einer Vorrichtung zur Bereitstellung ergänzter Plasmaprodukte für einen Patienten, der diese benötigt, umfassend:

(a) Mittel zur Einführung von Plasma eines Patienten in eine Bioreaktoruntereinheit des seriell verknüpften Bioreaktors aus Anspruch 21,
(b) Mittel, die mindestens einen Teil des Plasmas in einen radialen Fluss durch ein Zellkompartiment der Bioreaktoruntereinheit zwingen, um einen biotransformierten Ausfluss zu bilden,
(c) Mittel zur Einführung des biotransformierten Ausflusses in eine zweite Bioreaktoruntereinheit des Bioreaktors aus Anspruch 21,
(d) Mittel, die mindestens einen Teil des biotransformierten Ausflusses in einen radialen Fluss durch ein Zellkompartiment der zweiten Bioreaktoruntereinheit zwingen, um ergänztes Plasma zu bilden, und
(e) Mittel zur Rückführung des ergänzten Plasmas in das Kreislaufsystem des Patienten.

**27.** Multi-koaxialer Hohlfaser-Bioreaktor, umfassend:

(a) ein Gehäuse, das eine Innenseite umfasst, und
(b) ein Modul von Hohlfasern, umfassend: mindestens drei koaxiale semipermeable Hohlfasern, einschließlich einer innersten Faser mit einer Innenseite, wobei die Innenseite ein erstes Kompartiment definiert, das mindestens eine innerste Einlassöffnung und mindestens eine innerste Auslassöffnung umfasst, eine Vielzahl Kompartimente, wobei jedes Kompartiment durch einen jeweiligen annularen Zwischenraum zwischen benachbarten Fasern der mindestens drei Hohlfasern definiert ist, einschließlich mindestens einer äußeren Einlassöffnung und mindestens einer äußeren Auslassöffnung,
wobei jedes Kompartiment ein Mittel zur Fließverbindung zwischen dem jeweiligen annularen Zwischenraum, der jeweiligen äußeren Einlassöffnung und der jeweiligen äußeren Auslassöffnung umfasst und worin einer der annularen Zwischenräume eukaryotische Zellen enthält, und
(c) ein äußerstes Kompartiment, das durch einen Zwischenraum zwischen der Außenseite der äußersten Faser der mindestens drei Hohlfasern und der Innenseite des Gehäuses definiert ist und mindestens eine äußerste Einlassöffnung und mindestens eine äußerste Auslassöffnung umfasst,
(d) wobei das Gehäuse mindestens einen Einlassverteiler und mindestens einen Auslassverteiler für jedes der Kompartimente umfasst, und
(e) worin mindestens eines der Kompartimente eukaryotische Zellen enthält.

**28.** Verwendung des Bioreaktors nach Anspruch 27 zum Aufbau einer Vorrichtung für die Zellkultur, umfassend: Mittel zur Einführung lebensfähiger Zellen in ein Kompartiment des Bioreaktors und Mittel zur Durchleitung von Nahrungsmittelmedium durch koaxiale benachbarte Hohlfasern.

**Revendications**

**1.** Bioréacteur comprenant :

(a) un logement comportant un côté interne comprenant : un moyen d'introduction des gaz réalisé en une seule pièce avec le logement et un moyen d'évacuation des gaz réalisé en une seule pièce avec le logement ;
(b) un réseau de plusieurs modules de fibres creuses, disposé à l'intérieur du logement, chaque module comprenant :

(i) plusieurs fibres creuses coaxiales, chacune possédant un côté interne et un côté externe, englobant une fibre creuse située le plus à l'intérieur et une fibre creuse située le plus à l'extérieur ;
(ii) plusieurs compartiments comprenant : un premier compartiment défini par le côté interne de la fibre creuse située le plus à l'intérieur ; et
(iii) au moins un compartiment supplémentaire défini par un espace annulaire respectif ménagé entre des fibres adjacentes parmi lesdites plusieurs fibres creuses coaxiales ; et

(c) un compartiment situé le plus à l'extérieur défini par un espace ménagé à l'intérieur du côté interne du logement, qui n'est pas occupé par lesdits plusieurs modules ;

(d) le premier compartiment, ledit au moins un compartiment supplémentaire et le compartiment situé le plus à l'extérieur comprenant chacun en outre au moins un orifice d'entrée et au moins un orifice de sortie ; et

(e) le logement comprenant en outre :

(i) au moins un collecteur d'entrée et au moins un collecteur de sortie pour le premier compartiment ; et
(ii) au moins un collecteur d'entrée et au moins un collecteur de sortie pour chaque compartiment supplémentaire.

2. Bioréacteur selon la revendication 1, dans lequel les fibres creuses sont des fibres semi-perméables.

3. Bioréacteur selon la revendication 2, dans lequel les fibres creuses comprennent une matière choisie parmi le groupe constitué par la polysulfone, le polypropylène, le nylon, le polyester, le polytétrafluoréthylène, l'acétate de cellulose et des esters mixtes de cellulose.

4. Bioréacteur selon la revendication 1, dans lequel le bioréacteur comprend en outre au moins $10^9$ cellules.

5. Bioréacteur selon la revendication 4, dans lequel les cellules sont des cellules hépatiques.

6. Bioréacteur selon la revendication 5, dans lequel les cellules hépatiques sont choisies parmi le groupe constitué par des cellules hépatiques porcines et des cellules hépatiques humaines.

7. Bioréacteur selon la revendication 1, dans lequel ledit au moins un collecteur comprend en outre un distributeur de l'écoulement.

8. Bioréacteur selon la revendication 7, dans lequel ledit au moins un compartiment comprend en outre une matrice extracellulaire.

9. Bioréacteur selon la revendication 1, dans lequel ledit au moins un espace annulaire s'élève d'environ 0,2 mm à environ 0,8 mm.

10. Bioréacteur selon la revendication 1, dans lequel le bioréacteur est stérilisé à l'aide d'un moyen choisi parmi le groupe constitué par un passage à l'autoclave, un traitement avec de l'oxyde d'éthylène et une exposition à un rayonnement gamma.

11. Bioréacteur selon la revendication 1, dans lequel la fibre creuse située le plus à l'intérieur possède une longueur d'environ 2 cm à environ 50 cm.

12. Bioréacteur selon la revendication 1, dans lequel le logement possède une première extrémité et une deuxième extrémité, chaque orifice d'entrée et chaque orifice de sortie étant disposés à la première extrémité du logement.

13. Bioréacteur selon la revendication 1, comprenant en outre des microfibres essentiellement parallèles aux modules de fibres creuses.

14. Bioréacteur selon la revendication 13, dans lequel les microfibres comprennent en outre au moins un orifice d'entrée d'aération et au moins un orifice de sortie d'aération.

15. Bioréacteur selon la revendication 1, dans lequel au moins une fibre creuse coaxiale est saturée avec du perfluorocarbone.

16. Bioréacteur selon la revendication 1, dans lequel au moins une fibre creuse coaxiale possède une grosseur de pore inférieure à $1 \times 10^{-6}$ m.

17. Bioréacteur selon la revendication 1, dans lequel au moins une fibre creuse coaxiale possède une grosseur de pore inférieure à $0,1 \times 10^{-6}$ m.

18. Bioréacteur selon la revendication 1, dans lequel au moins une fibre creuse coaxiale possède une grosseur de pore inférieure à 0,05 x 10$^{-6}$ m.

19. Bioréacteur selon la revendication 1, dans lequel au moins un compartiment comprend en outre des cellules en mélange avec une matrice extracellulaire.

20. Utilisation du bioréacteur selon la revendication 1 pour le montage d'un dispositif pour acheminer des produits de biosynthèse cellulaire à un patient qui en a besoin, le dispositif comprenant : un moyen pour pomper une solution d'alimentation intraveineuse à travers un compartiment dudit bioréacteur ; un moyen pour récolter le produit de sortie ; et un moyen pour acheminer ledit produit de sortie par voie intraveineuse au patient.

21. Bioréacteurs montés en série comprenant plusieurs sous-unités de bioréacteurs, chaque sous-unité de bioréacteur comprenant :

   (a) un logement comportant un côté interne comprenant : un moyen d'introduction des gaz réalisé en une seule pièce avec la logement et un moyen d'évacuation des gaz réalisé en une seule pièce avec le logement ;
   (b) un réseau de plusieurs modules de fibres creuses, disposé à l'intérieur du logement, chaque module comprenant :

      (i) plusieurs fibres creuses coaxiales, chacune possédant un côté interne et un côté externe, englobant une fibre creuse située le plus à l'intérieur et une fibre creuse située le plus à l'extérieur ;
      (ii) plusieurs compartiments comprenant : un premier compartiment défini par le côté interne de la fibre creuse située le plus à l'intérieur ; et au moins un compartiment supplémentaire défini par un espace annulaire respectif ménagé entre des fibres adjacentes parmi lesdites plusieurs fibres creuses coaxiales ; et

   (c) un compartiment situé le plus à l'extérieur défini par un espace ménagé à l'intérieur du côté interne du logement, qui n'est pas occupé par lesdits plusieurs modules ;
   (d) le premier compartiment, ledit au moins un compartiment supplémentaire et le compartiment situé le plus à l'extérieur comprenant chacun en outre au moins un orifice d'entrée et au moins un orifice de sortie ; et
   (e) le logement comprenant en outre :

      (i) au moins un collecteur d'entrée et au moins un collecteur de sortie pour le premier compartiment ; et
      (ii) au moins un collecteur d'entrée et au moins un collecteur de sortie pour chaque compartiment supplémentaire ; et

   (f) au moins un compartiment d'une sous-unité de bioréacteur monté en série à au moins un compartiment d'au moins une autre sous-unité de bioréacteur.

22. Bioréacteur selon la revendication 21, dans lequel chaque sous-unité de bioréacteur comprend en outre au moins 10$^9$ cellules.

23. Bioréacteur selon la revendication 22, dans lequel les cellules sont des cellules hépatiques.

24. Bioréacteur selon la revendication 23, dans lequel les cellules hépatiques sont choisies parmi le groupe constitué par des cellules hépatiques humaines et des cellules hépatiques porcines.

25. Bioréacteur selon la revendication 22, dans lequel ledit au moins un compartiment de chaque sous-unité de bioréacteur comprend en outre une matrice extracellulaire.

26. Utilisation des bioréacteurs montés en série selon la revendication 21 pour le montage d'un dispositif pour acheminer des produits plasmatiques suppléés à un patient qui en a besoin, le dispositif comprenant :

   (a) un moyen pour introduire le plasma d'un patient dans une sous-unité de bioréacteur des bioréacteurs montés en série selon la revendication 21 ;
   (b) un moyen pour forcer au moins une portion du plasma à s'écouler en direction radiale à travers un compartiment cellulaire de la sous-unité de bioréacteur pour former un effluent soumis à une biotransformation ;
   (c) un moyen pour introduire l'effluent soumis à une biotransformation dans une deuxième sous-unité de bioréacteur des bioréacteurs selon la revendication 21 ;

(d) un moyen pour forcer au moins une portion de l'effluent soumis à une biotransformation à s'écouler en direction radiale à travers un compartiment cellulaire de la deuxième sous-unité de bioréacteur pour obtenir un plasma suppléé ; et

(e) un moyen pour renvoyer le plasma suppléé au système circulatoire du patient.

27. Bioréacteur du type à multiples fibres creuses coaxiales, comprenant :

(a) un logement comprenant un côté interne ; et

(b) un module de fibres creuses comprenant : au moins trois fibres creuses coaxiales semi-perméables, englobant une fibre située le plus à l'intérieur possédant un côté interne, le côté interne définissant un premier compartiment qui comprend au moins un orifice d'entrée situé le plus à l'intérieur et au moins un orifice de sortie situé le plus à l'intérieur ; plusieurs compartiments, chaque compartiment étant défini par un espace annulaire respectif ménagé entre des fibres adjacentes parmi lesdites au moins trois fibres creuses, englobant au moins un orifice d'entrée externe et au moins un orifice de sortie externe ;

chaque compartiment comprenant un moyen de communication par écoulement entre l'espace annulaire respectif, l'orifice d'entrée externe respectif et l'orifice de sortie externe respectif, et un des espaces annulaires contenant des cellules eucaryotes ; et

(c) un compartiment situé le plus à l'extérieur défini par un espace ménagé entre le côté externe de la fibre située le plus à l'extérieur parmi lesdites au moins trois fibres creuses, et le côté interne du logement, et comprenant au moins un orifice d'entrée situé le plus à l'extérieur et au moins un orifice de sortie situé le plus à l'extérieur ;

(d) le logement comprenant au moins un collecteur d'entrée et au moins un collecteur de sortie pour chacun des compartiments ; et

(e) au moins un des compartiments contenant des cellules eucaryotes.

28. Utilisation du bioréacteur selon la revendication 27 pour le montage d'un dispositif destiné à une culture cellulaire, le dispositif comprenant :

un moyen pour introduire des cellules viables dans un compartiment dudit bioréacteur ; et

un moyen pour faire passer un milieu nutritif à travers des fibres creuses adjacentes disposées en position coaxiale.

FIG. 1A

102     RADIAL     106

100%

↕ 124

0%

0        0.2        0.4

◄── 126 ──►
(mm)

# FIG. 1B

AXIAL

0%     ◄── 128 ──►     100%

0 mm

↕

130 (mm)     134

132

25 mm

# FIG. 1C

FIG. 2B

FIG. 2A

FIG. 3A

FIG. 3B

FIG. 3C

116    116

308

302

308

216    216

308

304

308

214    214

308

306

308

212

# FIG. 3D

FIG. 3E

FIG. 4

EP 1 185 612 B1

## FIG. 5

FIG. 6A

218

610

302

FIG. 6B

606

602

218

302

FIG. 6D

612

614

218

302

FIG. 6C

216

624

620

304

FIG. 6E

216

304

FIG. 6F

622

624

304

FIG. 6G

636

326

FIG. 6H

FIG. 6I

FIG. 6J

FIG. 6K

FIG. 6L

FIG. 6M

FIG. 6N

FIG. 7

FIG. 8

FIG. 9

FIG. 10B

FIG. 10C

FIG. 10A

FIG. 11A

FIG. 11B

FIG. 11C

S20

INPUT APPARATUS AND CELL PARAMETERS

S22

INITIAL $\triangle P$

S24

HYDROSTATIC
PRESSURE
WITHIN LIMITS

YES

NO

S26

ADJUST $\triangle P$

S30

MEASURE Q

S28

YES

HYDROSTATIC
PRESSURE
WITHIN LIMITS

NO

S32

SHEAR FORCES
WITHIN LIMITS

NO

S34

APPLY FORMULA II
FOR NEW VALUES OF
$K_1$ AND/OR $K_2$

YES

S38

PROLONGED CELL SURVIVAL

FIG. 12

49

FIG. 13A

FIG. 13B

FIG. 14

EP 1 185 612 B1

1502

304

302

202

416

1504

1506

FIG. 15